# EUROPEAN PATENT APPLICATION

(11) **EP 2 749 277 A2**
(43) Date of publication of application: **02.07.2014**
(21) Application number: 14162108.6
(22) Date of filing: 17.02.2012
(51) Int. Cl.: A61K 31/138, A61K 31/40, A61K 31/57, A61K 31/58, A61P 11/06, A61P 11/00

(54) **Combination of glycopyrronium and vilanterol**

(30) Priority: 17.02.2011 IN MM04462011; 11.03.2011 IN MM06942011; 28.03.2011 IN MM09532011; 19.05.2011 IN MM15352011; 19.05.2011 IN MM15342011; 31.05.2011 IN MM16132011; 07.07.2011 IN MM19652011; 07.07.2011 IN MM19662011
(62) Divisional of application: 12707369.0
(71) Applicant: Cipla Limited, Mumbai - 400 008 (IN)
(72) Inventor: Malhotra, Geena, 400 010 Mumbai (IN); Purandare, Shrinivas Madhukar, 400 031 Mumbai (IN)
(74) Representative: King, Lawrence

(57) **Abstract**

The present invention relates to pharmaceutical compositions for inhalation comprising glycopyrronium in combination with vilanterol and optionally fluticasone; to a process for preparing such compositions and to the use of such compositions for the prevention and/or treatment of respiratory, inflammatory or obstructive airway disease.

## Description

### Field of Invention

The present invention relates to pharmaceutical compositions for inhalation which comprise one or more bronchodilators and optionally an inhaled corticosteroid. There is also provided a process for preparing such compositions and the use thereof in the treatment and/or prevention of respiratory, inflammatory or obstructive airway disease, particularly chronic obstructive pulmonary disease.

### Background of Invention

Chronic obstructive pulmonary disease (COPD) is a severe respiratory condition that is increasing its prevalence worldwide. In India, the estimated prevalence is about 12.36 million. It is currently the fourth leading cause of death in the UK & US, and predicted to rank third in the global impact of disease by the year 2020.

COPD is a preventable and treatable disease state characterized by air flow limitation that is not fully reversible. The airflow obstruction is usually progressive and associated with an abnormal inflammatory response of the lungs to noxious particles or gases, primarily caused by cigarette smoking. Although COPD affects the lungs it also produces significant systemic consequences. COPD is associated with mucus hyper secretion, emphysema, bronchiolitis.

The major goals of COPD therapy include smoking cessation, relief of symptoms, improvement in physiological functions and limiting complications, such as abnormal gas exchange and exacerbation of disease. However, an integrated approach to the treatment of COPD, involves a combination of healthcare maintenance such as smoking cessation, avoidance of indoor, outdoor pollutants and allergens, and avoidance of occupational exposure to allergens, use of drugs and supplemental therapies in a step-wise fashion as the disease progresses.

Currently, therapy for the treatment or prevention of COPD and asthma includes the use of one or more long acting bronchodilators and an inhaled corticosteroid (ICS).

Inhaled bronchodilators are the foundation in the therapy of COPD because of their capacity to alleviate symptoms, decrease exacerbations of disease and improve quality of life. These drugs also improve airflow limitation and hyperinflation, thereby decreasing the work of breathing and improving exercise tolerance. In addition, bronchodilators may reduce respiratory muscle fatigue and improve mucociliary clearance.

More specifically, the choice of bronchodilators includes beta₂-agonists and anticholinergics. Further, beta₂-agonists can be short acting for immediate relief, or long acting for long term prevention of asthma symptoms.

Long acting beta₂-agonists (LABAs) improve lung function, reduce symptoms and protect against exercise-induced dyspnea in patients with asthma and COPD. LABAs induce bronchodilation by causing prolonged relaxation of airway smooth muscle. In addition to prolonged bronchodilation, LABAs exert other effects such as inhibition of airway smooth-muscle cell proliferation and inflammatory mediator release, as well as non smooth-muscle effects, such as stimulation of mucociliary transport, cytoprotection of the respiratory mucosa and attenuation of neutrophil recruitment and activation.

Also, use of a LABA reduces the frequency of drug administration. Commercially available LABAs include salmeterol and formoterol.

Anticholinergic agents also act as bronchodilators and are potential alternatives to beta agonists, particularly LABAs. However, anticholinergics can also be administered along with LABAs for the management of asthma. Anticholinergics act by competing with acetylcholine for the receptor sites at vagus nerve or nerve-muscle junctions. This prevents the transmission of reflexes that are induced by asthma stimuli.

Use of anticholinergics provides an advantage in elderly patients as the responsiveness of beta₂-agonists declines with old age. Further it would be advantageous to use anticholinergics in patients who are intolerant to the use of beta₂-agonists.

Even though it is known that beta₂-agonists provide a symptomatic relief in bronchoconstriction, another component of COPD, which is inflammation, requires a separate treatment such as with steroids. Most of the inhaled corticosteroids need to be administered in multiple dosage regimens.

Corticosteroids exhibit inhibitory effects on inflammatory cells and inflammatory mediators involved in the pathogenesis of respiratory disorders such as COPD. Treatment with a corticosteroid/glucocorticoid is considered one of the most potent and effective therapies currently available for COPD.

However, the use of corticosteroids has been limited due to potential side effects associated with their use, including suppression of the Hypothalamic-Pituitary-Adrenal (HPA) axis, adverse effects on bone growth in children and on bone density in the elderly, ocular complications (cataract formation and glaucoma) and skin atrophy.

Commercially available corticosteroids include beclomethasone, budesonide, fluticasone, mometasone, ciclesonide and triamcinolone.

Currently, there are several commercially available pharmaceutical compositions for inhalation comprising combinations of LABA and inhaled corticosteroid (ICS). Examples of such combinations for the treatment of asthma and chronic obstructive pulmonary disease (COPD) are salmeterol/fluticasone propionate (Advair® diskus®, Advair® HFA), and formoterol fumarate dehydrate/budesonide (Symbicort®).

Thus combination therapy of a bronchodilator with an ICS improves pulmonary efficiency, reduces inflammatory response and provides symptomatic relief as compared to higher doses of ICS alone in patients affected by respiratory disorders such as COPD. The selection of a specific bronchodilator and ICS plays a very important role in formulation of fixed dose combination therapies.

Further, combination therapy reduces the cost and also provides control of respiratory disorders. Reducing the dose frequency to the minimum is a main step in simplifying COPD management for improving patient adherence to the therapy.

US2009088408 discloses pharmaceutical compositions of anticholinergics, corticosteroids and betamimetics and their use in the treatment of respiratory diseases. The examples of this application are inhalable powders or suspension aerosol compositions which contain tiotropium or ipratropium bromide.

US2005042174 discloses a combination of doses of a beta₂-agonist, an anticholinergic agent and an anti-inflammatory steroid.

WO2006105401 discloses anticholinergic in combination with a corticosteroid, and a long acting beta agonist, for simultaneous or sequential administration in the prevention or treatment of a respiratory, inflammatory or obstructive airway disease.

US2008279948 discloses a medicament comprising a beta₂-agonist, a glycopyrronium salt and mometasone furoate. The examples of this application contain the beta₂-agonist indacaterol maleate.

US2008286363 discloses a medicament comprising a beta₂-agonist (such as indacaterol maleate), a glycopyrronium salt and a corticosteroid. The examples of this application contain the corticosteroid 3-methyl-thiophene-2-carboxylic acid (6S,9R,10S,11S,13S,16R,17R)-9-chloro-6-fluoro-11-hydroxy-17-methoxycarbonyl-10,13,16-trimethyl-3-oxo-6,7,8,9,10,11,12,13,14,15,16,17-dodecahydro-3H-cyclopenta-[a]phenanthren-17-yl ester.

US2010166671 discloses a medicament comprising an antimuscarinic agent, a beta₂-agonist and a corticosteroid. The examples of this application contain glycopyrronioum, formoterol fumarate and mometasone furoate.

US7439393 discloses certain phenethanolamine derivatives for the treatment of respiratory diseases. The use of such compounds in combination therapy with other therapeutic agents is also disclosed.

US20080041369 discloses propellant-free aerosol formulations comprising *inter alia* olodaterol, a corticosteroid such as budesonide, beclomethasone or fluticasone and an anticholinergic such as tiotropium, oxitropium or ipratropium.

US20050239778 discloses medicament combinations comprising *inter alia* olodaterol and at least one other active substance, such as a steroid.

US20080317862 discloses medicaments comprising an antimuscarinic agent and a corticosteroid for the treatment of inflammatory or obstructive airways diseases. In particular, this application discloses aerosol compositions comprising glycopyrronium and mometasone furoate.

US20060069073 discloses a combination of glycopyrronium and one or more steroids as a second active substance.

WO2005110402 discloses medicaments comprising glycopyrrolate in combination with a beta₂-agonist such as indacaterol maleate.

WO2005074900 discloses a combination of an anticholinergic such as glycopyrronium and a long-acting beta-mimetic agent such as formoterol or salmeterol.

Thus, there is still a need to develop suitable combinations comprising a beta agonist, an anticholinergic agent and/or an inhaled corticosteroid that alleviate COPD.

Hence, there still exits a need to formulate pharmaceutical compositions comprising a beta agonist, an anticholinergic agent and an inhaled corticosteroid exhibiting reduced side effects and which can be administered once a day.

### Objects of the Invention

The object of the present invention is to provide pharmaceutical compositions for inhalation comprising one or more bronchodilators and an inhaled corticosteroid for administration in the prevention or treatment of respiratory, inflammatory or obstructive airway disease.

Another object of the present invention is to provide pharmaceutical compositions for inhalation comprising one or more bronchodilators and an inhaled corticosteroid for once daily administration for the prevention or treatment of respiratory, inflammatory or obstructive airway disease.

Yet another object of the present invention is to provide a process for preparing the pharmaceutical compositions comprising one or more bronchodilators and an inhaled corticosteroid for administration in the prevention or treatment of respiratory, inflammatory or obstructive airway disease.

A further object of the present invention is to provide a method for prophylaxis or treatment of COPD which comprises administering pharmaceutical compositions comprising one or more bronchodilators and an inhaled corticosteroid.

### Summary of the Invention

According to a first aspect of the present invention, there is provided a pharmaceutical composition comprising glycopyrrolate and a beta₂-agonist.

Preferably the composition further comprises one or more inhaled corticosteroids.

According to a second aspect of the present invention, there is provided a pharmaceutical composition comprising glycopyrrolate and vilanterol.

According to a third aspect of the present invention, there is provided a pharmaceutical composition comprising glycopyrrolate and olodaterol.

According to a fourth aspect of the present invention, there is provided a pharmaceutical composition comprising glycopyrrolate and carmoterol.

According to a fifth aspect of the present invention, there is provided a pharmaceutical composition comprising glycopyrrolate, olodaterol and fluticasone, especially an ester of fluticasone, in particular fluticasone furoate.

According to a sixth aspect of the present invention, there is provided a pharmaceutical composition comprising glycopyrrolate, olodaterol and mometasone, especially an ester of mometasone, in particular mometasone furoate.

According to a seventh aspect of the present invention, there is provided a pharmaceutical composition comprising glycopyrrolate, vilanterol and fluticasone, especially an ester of fluticasone, in particular fluticasone furoate.

According to a eighth aspect of the present invention, there is provided a pharmaceutical composition comprising glycopyrrolate, fomoterol and fluticasone, especially an ester of fluticasone, in particular fluticasone furoate.

According to a ninth aspect of the present invention, there is provided a pharmaceutical composition comprising glycopyrrolate, indacetrol and fluticasone, especially an ester of fluticasone, in particular fluticasone furoate.

According to a tenth aspect of the present invention, there is provided a process for preparing the pharmaceutical compositions described above.

According to a eleventh aspect of the present invention, there is provided a method for prophylaxis or treatment of asthma, COPD or a related respiratory disorder which comprises administering a pharmaceutical compositions described above.

According to a twelfth aspect of the present invention there is provided a use in treating disorders or conditions that respond to, or are prevented, ameliorated or eliminated by, the administration of pharmaceutical compositions described above.

### Detailed Description of the Invention

As discussed above, the selection of a specific beta₂-agonist, anticholinergic agent and inhaled corticosteroid (ICS) plays a very important role in formulation of fixed dose combinations.

The present invention thus provides pharmaceutical compositions for inhalation comprising or consisting of glycopyrrolate, a beta₂-agonist, and an inhaled corticosteroid.

In one embodiment, there is provided a pharmaceutical composition for inhalation comprising or consisting of:
(a) glycopyrrolate;
(b) a beta₂-agonist selected from the group consisting of carmoterol, formoterol, indacaterol, olodaterol, vilanterol; and, optionally, when the LABA is selected from formoterol, indacaterol, olodaterol, vilanterol;
(c) an inhaled corticosteroid (ICS) selected from the group consisting of fluticasone, mometasone;
preferably wherein (a), (b) and (c) are formulated for simultaneous, separate or sequential administration; and provided that the composition does not comprise glycopyrrolate, mometasone furoate and indacaterol maleate or formoterol fumarate.

A particularly preferred pharmaceutical composition of the present invention comprises, or consists of, (a) glycopyrrolate (b) indacaterol and (c) fluticasone (especially fluticasone furoate).

A further particularly preferred pharmaceutical composition of the present invention comprises, or consists of, (a) glycopyrrolate, (b) formoterol and (c) fluticasone (especially fluticasone furoate).

A further particularly preferred pharmaceutical composition of the present invention comprises, or consists of, (a) glycopyrrolate (b) vilanterol and (c) fluticasone (especially fluticasone furoate).

A further particularly preferred pharmaceutical composition of the present invention comprises, or consists of, (a) glycopyrrolate, (b) olodaterol and (c) fluticasone (especially fluticasone furoate).

A still further particularly preferred pharmaceutical composition of the present invention comprises, or consists of, (a) glycopyrrolate, (b) olodaterol and (c) mometasone.

In an alternative preferred embodiment of the invention, there is provided a pharmaceutical composition comprising or consisting of glycopyrrolate and a beta₂-agonist.

In a still further preferred embodiment of the invention, there is provided a pharmaceutical composition comprising or consisting of (a) glycopyrrolate; and (b) a beta₂-agonist selected from the group consisting of carmoterol, olodaterol, vilanterol; preferably wherein (a) and (b) are formulated for simultaneous, separate or sequential administration.

A particularly preferred pharmaceutical composition of the present invention comprises, or consists of, (a) glycopyrrolate and (b) vilanterol.

A further particularly preferred pharmaceutical composition of the present invention comprises, or consists of, (a) glycopyrrolate and (b) olodaterol.

A still further particularly preferred pharmaceutical composition of the present invention comprises, or consists of, (a) glycopyrrolate and (b) carmoterol.

Our inventors have found that the above-mentioned pharmaceutical compositions are effective for treating inflammatory and/or obstructive diseases of the respiratory tract, particularly asthma or chronic obstructive pulmonary disease (COPD).

Furthermore, the pharmaceutical compositions of the present invention advantageously provide a rapid onset of action, longer duration of action and improved control of obstructive or inflammatory airway diseases, or reduction in the exacerbations of the diseases.

Also, the pharmaceutical compositions of the present invention advantageously reduce the risk of undesirable side effects as compared to the repeated exposure of the steroid alone involved in the treatment of inflammatory or obstructive airways diseases.

Another advantage of the pharmaceutical compositions of the present invention is that the invention facilitates the treatment of an obstructive and inflammatory airway disease with a single medicament.

Further the pharmaceutical compositions of the present invention provide for the administration of combination therapies by use of a single inhaler for patients who currently have to make use of multiple inhalers. By way of example, patients may administer pharmaceutical compositions of the present invention from a single inhaler instead of administering from three different inhalers, one for corticosteroid, one for anticholinergic and one for a long acting beta₂-agonist. This is particularly important in case of elderly patients who may get confused between the inhalers and who also suffer from several other medical conditions such as heart disease and arthritis, and are receiving multiple other medications.

In a preferred embodiment, the pharmaceutical compositions of the present invention are formulated for once daily administration.

The pharmaceutical compositions of the present invention comprise glycopyrrolate. The word "glycopyrrolate" can be interchangeably used with "glycopyrronium". Glycopyrrolate belongs to the class of quaternary ammonium anticholinergic drugs and antagonizes the neurotransmitter acetylcholine at its muscarinic receptors. This effect leads to a considerable smooth muscle relaxation resulting in a prolonged bronchodilating effect. More specifically it inhibits acetylcholine binding to M3 muscarinic receptors thereby inhibiting bronchoconstriction.

Glycopyrrolate is a quaternary ammonium salt. Suitable counter ions are pharmaceutically acceptable counter ions including, for example, fluoride, chloride, bromide, iodide, nitrate, sulfate, phosphate, formate, acetate, trifluoroacetate, propionate, butyrate, lactate, citrate, tartrate, malate, maleate, succinate, benzoate, p-chlorobenzoate, diphenyl-acetate or triphenylacetate, o-hydroxybenzoate, p-hydroxybenzoate, 1-hydroxynaphthalene-2-carboxylate, 3-hydroxynaphthalene-2-carboxylate, methanesulfonate and benzenesulfonate. A particularly preferred salt of glycopyrrolate is the bromide salt thereof. The bromide salt of glycopyrrolate is chemically known as {3-[(Cyclopentyl-hydroxyphenylacetyl) oxy]-1,1-dimethylpynrolidinium bromide}.

Glycopyrrolate has two centers of asymmetry (chiral centers), and can exist in four stereoisometric forms namely (3R, 2'R)-, (3S, 2'R)-, (3R, 2'S)- and (3S, 2'S), i. e. , two enantiomeric pairs of diastereomers. The two di- astereomer pairs have been separated from one another. Commercially available formulations of glycopyrrolate contain both the (R, S)-glycopyrrolate and (S, R)-glycopyrrolate enantiomers.

Glycopyrrolate is currently available marketed in the form of oral tablets for adjunctive therapy in the treatment of peptic ulcer, as an injectable for therapy in the treatment of peptic ulcer and as a preoperative antimuscarinic to reduce secretions and as a capsule for reducing chronic severe drooling in patients aged between 3 to 16 years with neurologic conditions associated with problem drooling.

Glycopyrrolate also prevent the effects resulting from passage of impulses through the parasympathetic nerves. This action results from their ability to inhibit the action of the neurotransmitter acetylcholine by blocking its binding to muscarinic cholinergic receptors. Further, inhaled glycopyrrolate exhibits low systemic absorption, and therefore is not associated with typical systemic antimuscarinic adverse effects.

According to the present invention, glycopyrrolate may be present in an amount of from about 50mcg to about 200mcg.

Bronchodilators used according to the present invention may be beta-agonists and/or anticholinergics. According to the present invention, beta agonists may comprise, one or more, short acting beta agonist(s), long acting beta agonist(s) and/or ultra long acting beta agonist(s).

In addition to glycopyrrolate, the pharmaceutical compositions of the present invention further comprise a beta₂-agonist, preferably selected from the group comprising carmoterol, formoterol, indacaterol, olodaterol, vilanterol.

Carmoterol is chemically known as 8-hydroxy-5 - (1-hydroxy-2-(N-(2-(4-methoxy:phenyl) -1-methyl:ethyl) amino)ethyl)-2 (1H)-quinolinone. Carmoterol is a long acting beta₂-agonist characterized by having a rapid onset of action, prolonged duration of action and also having a high selectivity towards the beta₂ adrenoreceptor. Furthermore, carmoterol is more potent than other LABAs such as formoterol and salmeterol. A particularly preferred pharmaceutically acceptable salt of carmoterol is carmoterol hydrochloride. According to the present invention, carmoterol may be present in an amount of from about 1mcg to about 4mcg.

Formoterol is chemically known as (±)-2-hydroxy-5-[(1RS)-1-hydroxy-2-[[(1RS)-2-(4-methoxyphenyl)-1methylethyl]-amino] ethyl] formanilide. Formoterol is a selective LABA. Formoterol exhibits a quick onset of action (1-3 minutes) which helps to achieve an immediate therapeutic response. Furthermore formoterol exhibits a long duration of action of more than 12 hours. A particularly preferred pharmaceutically acceptable ester of formoterol is formoterol fumarate. A particularly preferred pharmaceutically acceptable ester of formoterol is formoterol fumarate dihydrate. According to the present invention, formoterol may be present in an amount of from about 12 to about 24mcg, preferably about 24mcg.

Indacaterol is chemically known as (*R*)-5-[2-[(5,6-Diethyl-2,3-dihydro-1*H-*inden-2-yl)amino]-1-hydroxyethyl]-8-hydroxyquinolin-2(1*H*)-one is a ultra-long acting beta₂-agonist. Indacaterol has a fast onset of action which is similar to that of formoterol and faster than that of salmeterol. Furthermore, indacaterol exhibits a longer duration of action than salmeterol as well as has greater cardiovascular safety margin as compared to salmeterol and formoterol. A particularly preferred pharmaceutically acceptable salt of indacaterol is indacterol maleate. According to the present invention, indacaterol may be present in an amount of from about 25mcg to about 800mcg.

Olodaterol is chemically known as 6-hydroxy-8-[(1R)-1-hydroxy-2-[[2-(4-methoxyphenyl)-1,1-dimethylethyl]amino ethyl]-2H-1,4-benzoxazin-3(4H)- one. A particularly preferred pharmaceutically acceptable salt of olodaterol is olodaterol hydrochloride monohydrate. According to the present invention, olodaterol may be present in an amount of from about 3mcg to about 50mcg.

Vilanterol is chemically known as 4-{(1R)-2-[(6-{2-[(2,6-dichlorobenzyl)oxy]ethoxy}hexyl)amino]-1-hydroxyethyl}-2-(hydroxymethyl)phenol is a long acting beta₂-agonist. A particularly preferred pharmaceutically acceptable salt of vilanterol is vilanterol trifenatate. According to the present invention, vilanterol may be present in an amount of from about 3mcg to about 50mcg.

In addition to glycopyrrolate and a beta₂-agonist, the pharmaceutical compositions of the present invention may also comprise a corticosteroid; preferably selected from the group consisting of mometasone, fluticasone.

Fluticasone is currently commercially available as a furoate salt and a propionate salt. Fluticasone furoate is a novel corticosteroid which substantially overcomes the potential side effects that are generally produced by the use of conventional corticosteroids. Moreover fluticasone furoate exhibits a 1.7 times higher binding affinity for the human glucocorticoid receptor as compared to that of fluticasone propionate and also provides prolonged protection up to 26 hours against airway hyperresponsiveness as compared to fluticasone propionate. Fluticasone furuoate has a longer duration of action with an elimination half life of 15.1 hrs.

Fluticasone furoate is a synthetic fluorinated corticosteroid that has been developed as an intranasal treatment for patients with symptoms of rhinitis and has an enhanced affinity towards the glucocorticoid receptor. Further, fluticasone furoate has greater potency than other clinically used corticosteroids such as mometasone furoate, budesonide, fluticasone propionate, ciclesonide for the glucocorticoid receptor and against the proinflammatory transcription factors nuclear factor κB (NF-κB), activation protein-1, and tumor necrosis factor- induced interleukin-8 cytokine production. Chronic inflammation which is commonly associated with asthma is managed by fluticasone furoate.

Particularly preferred pharmaceutically acceptable esters of fluticasone are fluticasone furoate and fluticasone propionate, most preferably fluticasone furoate. According to the present invention, fluticasone furoate may be present in an amount of from about 25mcg to about 800mcg.

Mometasone furoate is chemically known as (11[β], 16[α])-9, 21-dichloro-17-[(2-furanylcarbonyl) oxy]-11-hydroxy-16-methylpregna-1,4-diene-3,20-dione. Mometasone furoate is a synthetic 17-heterocyclic corticosteroid and exhibits a long duration of action A particularly preferred pharmaceutically acceptable ester of mometasone is mometasone furoate. According to the present invention, mometasone furoate may be present in an amount of from about 400mcg to about 800mcg.

As used herein the terms "glycopyrronium", "glycopyrrolate", "fluticasone furoate", "mometasone furoare", "carmoterol", "olodaterol, "vilanterol', "formoterol" and "indacaterol" are used in broad sense to include not only "glycopyrronium", "glycopyrrolate" "fluticasone furoate" "mometasone furoare", "carmoterol", "olodaterol, "vilanterol', "formoterol" and "indacaterol" per se but also their pharmaceutically acceptable salts, pharmaceutically acceptable solvates, pharmaceutically acceptable hydrates, pharmaceutically acceptable enantiomers, pharmaceutically acceptable derivatives, pharmaceutically acceptable polymorphs, pharmaceutically acceptable prodrugs, etc.

In addition to active pharmaceutical ingredients, the pharmaceutical compositions of the present invention typically comprise one or more pharmaceutically acceptable excipients. The active ingredients may be used as separate formulations or as a single combined formulation. When combined in the same formulation, it will be appreciated that the active ingredients must be stable and compatible with each other and the other components of the formulation.

The pharmaceutical compositions of the present invention are formulated for inhalation and may therefore be administered by any suitable methods used for delivery of the drugs to the respiratory tract. For example, the composition of the present invention may be in the form of an aerosol composition, a nasal spray, nasal drops or an insufflation powder. Such aerosol compositions may be administered by any conventional means, for example using a metered dose inhaler (MDI), dry powder inhaler (DPI) or nebulizer.

The various dosage forms according to the present invention may comprise carriers/excipients suitable for formulating the same.

In one embodiment, the pharmaceutical compositions of the present invention are in a form suitable for administration by a MDI, for example, in the form of an aerosol composition. Such compositions may comprise one or more pharmaceutically acceptable excipients, in particular selected from the group of HFC/HFA propellants, co-solvents, bulking agents, non-volatile components, buffers/pH adjusting agents, surface active agents, preservatives, complexing agents, or combinations thereof.

Suitable propellants are those which, when mixed with the cosolvent(s), form a homogeneous propellant system in which a therapeutically effective amount of the medicament can be dissolved. The HFC/HFA propellant must be toxicologically safe and must have a vapor pressure which is suitable to enable the medicament to be administered via a pressurized MDI.

According to the present invention, the HFC/HFA propellants may comprise, one or more of 1,1,1,2-tetraffuoroethane (HFA-134(a)) and 1,1,1,2,3,3,3,-heptafluoropropane (HFA-227), HFC-32 (difluoromethane), HFC-143(a) (1,1,1-trifluoroethane), HFC-134 (1,1,2,2-tetrafluoroethane), and HFC-152a (1,1-diffuoroethane) or combinations thereof and such other propellants which may be known to the person having a skill in the art.

In the context of the present invention, the term "co-solvent" means any solvent which is miscible in the formulation in the amount desired and which, when added provides a formulation in which the medicament can be dissolved. The function of the co-solvent is to increase the solubility of the medicament and the excipients in the formulation.

According to the present invention, the co-solvent may comprise one or more of, C₂₋ C₆ aliphatic alcohols, such as, but not limited to, ethyl alcohol and isopropyl alcohol; glycols such as but not limited to propylene glycol, polyethylene glycols, polypropylene glycols, glycol ethers, and block copolymers of oxyethylene and oxypropylene; and other substances, such as, but not limited to, glycerol, polyoxyethylene alcohols, and polyoxyethylene fatty acid esters; hydrocarbons such as but not limited to n-propane, n-butane, isobutane, n-pentane, iso-pentane, neo-pentane, and n-hexane; and ethers such as but not limited to diethyl ether and combinations thereof.

Suitable surfactants which may be employed in an aerosol composition of the present invention include those which may serve to stabilize the solution formulation and improve the performance of valve systems of the metered dose inhaler. Preferred surfactants include one or more ionic and/or non- ionic surfactants. Examples of suitbale surfactants include, but are not limited to, oleic acid, sorbitan trioleate, lecithin, isopropylmyristate, tyloxapol, polyvinylpyrrolidone, polysorbates such as polysorbate 80, vitamin E-TPGS, and macrogol hydroxystearates such as macrogol-15-hydroxystearate and combinations thereof.

In the context of the present invention, the term "non-volatile component" refers to the suspended or dissolved constituents of the pharmaceutical composition that would remain after evaporation of the solvent(s) present.

According to the present invention, the non-volatile component may comprise one or more of monosaccharides such as, but not limited to, glucose, arabinose; disaccharides such as lactose, maltose; oligosaccharides and polysaccharides such as, but not limited to, dextrans; polyalcohol such as, but not limited to, glycerol, sorbitol, mannitol, xylitol; salts such as, but not limited to, potassium chloride, magnesium chloride, magnesium sulphate, sodium chloride, sodium citrate, sodium phosphate, sodium hydrogen phosphate, sodium hydrogen carbonate, potassium citrate, potassium phosphate, potassium hydrogen phosphate, potassium hydrogen carbonate, calcium carbonate and calcium chloride and combinations thereof.

Suitable bulking agents may be employed in the pharmaceutical compositions of the invention, in particular aerosol compositions that are intended for administration using an MDI. The bulking agent may comprise one or more of saccharides, including monosaccharides, disaccharides, polysaccharides and sugar alcohols such as arabinose, glucose, fructose, ribose, mannose, sucrose, terhalose, lactose, maltose, starches, dextran or mannitol and combinations thereof.

Suitable buffers or pH adjusting agents may be employed in the pharmaceutical compositions of the invention, in particular aerosol compositions that are intended for administration using an MDI. The buffer or the pH adjusting agent may comprise one or more of organic or inorganic acids such as, but not limited to, citric acid, ascorbic acid, hydrochloric acid, sulfuric acid, nitric acid, or phosphoric acid and combinations thereof.

Suitable preservatives may be employed in in the pharmaceutical compositions of the invention, in particular aerosol compositions that are intended for administration using an MDI, to protect the formulation from contamination with pathogenic bacteria. The preservative may comprise one or more of benzalkonium chloride, benzoic acid, benzoates such as sodium benzoate and such other preservatives which may be known to the person having a skill in the art and combinations thereof.

Suitable complexing agents may be employed in the pharmaceutical compositions of the invention, in particular aerosol compositions that are intended for administration using an MDI, capable of forming complex bonds. The complexing agent may comprise one or more of, but not limited to, sodium EDTA or disodium EDTA and combinations thereof.

In one embodiment, the pharmaceutical compositions of the present invention are in a form suitable for administration by a dry powder inhaler (DPI).

The pharmaceutically acceptable excipients suitable for dry powder inhalation according to the present invention may be selected from suitable carriers which include, but are not limited to, sugars such as glucose, saccharose, lactose and fructose, starches or starch derivatives, oligosaccharides such as dextrins, cyclodextrins and their derivatives, polyvinylpyrrolidone, alginic acid, tylose, silicic acid, cellulose, cellulose derivatives (for example cellulose ether), sugar alcohols such as mannitol or sorbitol, calcium carbonate, calcium phosphate, etc. lactose, lactitol, dextrates, , dextrose, maltodextrin, saccharides including monosaccharides, disaccharides, polysaccharides; sugar alcohols such as arabinose, ribose, mannose, sucrose, trehalose, maltose, dextran and combinations thereof.

In an alternative embodiment, the pharmaceutical compositions of the present invention are in a form suitable for administration by nebulization.

Nebulization therapy has an advantage over other inhalation therapy, since it is easy to use and does not require co-ordination or much effort. It also works much more rapidly than medicines taken by mouth. Such compositions may comprise suitable excipients such as one or more, but not limited to, tonicity agents, pH regulators, and chelating agents in a suitable vehicle.

Examples of suitable isotonicity-adjusting agents include sodium chloride, potassium chloride, zinc chloride, calcium chloride and mixtures thereof. Other isotonicity-adjusting agents may also include, but are not limited to, mannitol, glycerol, and dextrose and mixtures thereof.

The pH of pharmaceutical compositions of the invention may be adjusted by the addition of one or more pH regulators such as pharmacologically acceptable acids. Pharmacologically acceptable inorganic acids or organic acids may be used for this purpose. Examples of preferred inorganic acids include one or more acids selected from the group consisting of hydrochloric acid, hydrobromic acid, nitric acid, sulphuric acid and phosphoric acid and combinations thereof. Examples of particularly suitable organic acids include one or more acids selected from the group consisting of ascorbic acid, citric acid, malic acid, tartaric acid, maleic acid, succinic acid, fumaric acid, acetic acid, formic acid and propionic acid and combinations thereof.

Examples of suitable chelating agents for use in a pharmaceutical compositions of the invention include editic acid (EDTA) or a salt thereof, e.g. sodium EDTA or disodium EDTA dihydrate (sodium edetate), and mixtures of such compounds.

In addition to the excipients such as isotonicity-adjusting agents, pH regulators, chelating agents covered under nebulization therapy, the dosage forma as nasal spay and nasal drops may comprise thickening agents.

Examples of suitable thickening agents may for use in a pharmaceutical compositions of the invention include cellulose derivatives (for example cellulose ether) in which the cellulose-hydroxy groups are partially etherized with lower unsaturated aliphatic alcohols and/or lower unsaturated aliphatic oxyalcohols (for example methyl cellulose, carboxymethyl cellulose, hydroxypropylmethylcellulose), gelatin, polyvinylpyrrolidone, tragacanth, ethoxose (water soluble binding and thickening agents on the basis of ethyl cellulose), alginic acid, polyvinyl alcohol, polyacrylic acid, pectin and equivalent agents. Should these substances contain acid groups, the corresponding physiologically acceptable salts may also be used.

In addition to the aforementioned excipients, one or more anti-microbial preservative agents may also be added to the pharmaceutical compositions of the invention, in particular for multi-dose packages.

In an alternative embodiment, the composition according to the present invention may be included in one or more suitable containers provided with means enabling the application of the contained formulation to the respiratory tract.

Where the pharmaceutical compositions of the invention are in the form of a powder for inhalation and are intended to be administered by a DPI, it may be encapsulated in capsules of gelatin or HPMC, or in blisters. In an alternative embodiment, the dry powder may be contained as a reservoir either in a single dose or multi-dose dry powder inhalation device. In a further alternative embodiment, the powder for inhalation may be suspended in a suitable liquid vehicle and packed in an aerosol container along with suitable propellants or mixtures thereof. In still a further alternative embodiment, the powder for inhalation may be dispersed in a suitable gas stream to form an aerosol composition.

Where the pharmaceutical compositions of the invention are in the form of an aerosol composition for administration using an MDI, it may be packed in plain aluminium cans or SS (stainless steel) cans or any such cans suitable for MDI delivery. Some aerosol drugs tend to adhere to the inner surfaces, i.e., walls of the cans and valves, of the MDI. This can lead to the patient getting significantly less than the prescribed amount of the active agent upon each activation of the MDI. Such cans may be suitably treated to avoid any adherence of the active on the walls thereof using techniques known in the art, for example coating the inner surface of the container with a suitable polymer can reduce this adhesion problem. Suitable coatings include fluorocarbon copolymers such as FEP-PES (fluorinated ethylene propylene and polyethersulphone) and PFA-PES (perfluoroalkoxyalkane and polyethersulphone), epoxy and ethylene. Alternatively, the inner surfaces of the cans may be anodized, plasma treated or plasma coated.

Where the pharmaceutical compositions of the invention are in the form of nasal sprays and nasal drops for administration into the nasal passages it may be done by means of a dropper (or pipette) that includes a glass, plastic or metal dispensing tube. Fine droplets and sprays can be provided by an intranasal pump dispenser or squeeze bottle as well known in the art.

The pharmaceutical compositions of the present invention may further comprise, in addition to those pharmaceutically active ingredients detailed above, one or more active(s) selected from the group comprising of , antihistamines, antiallergics or leukotriene antagonists, or their pharmaceutically acceptable salts, solvates, tautomers, derivatives, enantiomers, isomers, hydrates, prodrugs or polymorphs thereof.

The pharmaceutical compositions of the present invention comprise glycopyrrolate, a beta₂-agonist and, optionally, a corticosteroid. These active ingredients are formulated for simultaneous, separate or sequential administration. When the active ingredients are administered sequentially, either glycopyrrolate the long acting beta₂-agonist, or where present, the corticosteroid, may be administered first. When administration is simultaneous, the active ingredients may be administered either in the same or different pharmaceutical compositions. Adjunctive therapy, i.e. where one active ingredient is used as the primary treatment and the other active ingredient(s) is/are used to assist that primary treatment is also an embodiment of the present invention.

According to a further embodiment of the invention, there is provided a product comprising (a) glycopyrrolate; (b) a beta₂-agonist selected from the group comprising carmoterol, olodaterol, vilanterol; as a combined preparation for simultaneous, separate or sequential use for treatment and /or prevention of respiratory, inflammatory or obstructive airway disease

According to a another embodiment of the invention, there is provided a product comprising (a) glycopyrrolate; (b) a beta₂-agonist selected from the group comprising olodaterol, vilanterol, formoterol, indacaterol (c) a corticosteroid selected from the group consisting of fluticasone, mometasone, as a combined preparation for simultaneous, separate or sequential use for treatment and /or prevention of respiratory, inflammatory or obstructive airway disease

Compositions for use according to the present invention may be presented in a pack or dispenser device which may contain one or more unit dosage forms containing the active ingredients. These may for example, comprise metal or plastic foil, such as a blister pack. Where compositions are intended for administration as two separate compositions these may be presented in the form of a twin pack.

Pharmaceutical compositions may also be prescribed in "patient packs" containing the whole course of treatment in a single package. The inclusion of a package insert has been shown to improve patient compliance with the prescribing physician's instructions. According to a further embodiment of the present invention, there is provided a patient pack comprising at least one active ingredient of the combination according to the invention and an information insert containing directions to use the combination of the invention. In one embodiment, the present invention provides a fixed dose combination.

The pharmaceutical compositions of the present invention may be conveniently presented in unit dosage form and may be prepared by any of the methods well known in the art. Suitable methods include the step of bringing into association the active ingredients with a carrier which constitutes one or more pharmaceutically acceptable excipients. In general, compositions may be prepared by uniformly and intimately bringing into association the active ingredients with one or more liquid carriers or finely divided solid carriers, or both. It will be appreciated that when the active ingredients are administered independently, each may be administered by a different means.

The present invention also provides a process to manufacture the compositions according to the present invention.

In one embodiment, the present invention provides a process of preparing pharmaceutical compositions for administration by a metered dose inhaler, which process comprises admixing a pharmaceutically acceptable carrier or excipient with one or more active pharmaceutical ingredients of the invention and a propellant, and thereafter transferring the composition to a suitable container, preferably a pre-crimped can.

In another embodiment, the invention provides a process of preparing a pharmaceutical compositions for administration by dry powder inhalation, which process comprises admixing of a pharmaceutically acceptable carrier or excipient with one or more active pharmaceutical ingredients of the invention and providing the composition as a dry powder.

In a further embodiment, the invention provides a process of preparing pharmaceutical compositions for administration by nebulisation, which process comprises dissolving the drugs, optionally chelating agents, osmotic/isotonicity adjusting agents and any other suitable ingredients in the vehicle and adjusting the pH using a suitable pH adjusting agent.

In a further embodiment, the invention also provides a method for the prevention and/or treatment of a respiratory, inflammatory or obstructive airway disease, in particular chronic obstructive pulmonary disease, in a mammal, such as a human, which method comprises administration of a therapeutically effective amount of pharmaceutical compositions according to the present invention.

The present invention also provides pharmaceutical compositions according to the present invention for use in preventing and/or treating disorders or conditions that respond to, or are prevented, ameliorated or eliminated by, the administration one or more bronchodilators and an inhaled corticosteroid (ICS), such as a respiratory, inflammatory or obstructive airway disease, in particular chronic obstructive pulmonary disease.

The following examples are for the purpose of illustration of the invention only and are not intended in any way to limit the scope of the present invention.

### Example 1

| **Sr. No.** | **Ingredients** | **Qty /Spray** |
|---|---|---|
| 1. | Fluticasone Furoate | 50 mcg |
| 2. | Glycopyrronium | 50 mcg |
| 3. | Indacaterol | 50 mcg |
| 4. | HFA134A OR HFA227 | q.s |

### Process:

1) Fluticasone furoate, Glycopyrronium and Tiotropium were homogenized with part quantity of HFA.
2) The suspension obtained in step 1 was transferred to the mixing vessel where remaining quantity of HFA was added.
3) The resulting suspension was mixed, recirculated and filled in into pre-crimped aluminum cans.

### Example 2

| **Sr. No.** | **Ingredients** | **Qty /Spray** |
|---|---|---|
| 1. | Fluticasone Furoate | 50 mcg |
| 2. | Glycopyrronium | 50 mcg |
| 3. | Indacaterol | 50 mcg |
| 4. | Lactose | 100% of the drug |
| 5. | HFA134A OR HFA227 | q.s. |

### Process:

1) Fluticasone furoate, Indacaterol and Glycopyrronium were homogenized with lactose and part quantity of HFA.
2) The suspension obtained in step 1 was transferred to the mixing vessel where remaining quantity of HFA was added.
3) The resulting suspension was mixed, recirculated and filled in into pre-crimped aluminum cans.

### Example 3

| **Sr. No.** | **Ingredients** | **Qty /Spray** |
|---|---|---|
| 1. | Fluticasone Furoate | 50 mcg |
| 2. | Glycopyrronium | 50 mcg |
| 3. | Indacaterol | 50 mcg |
| 4. | PEG400/1000 | 0.3% of total formulation |
| 5. | PVP K 25 | 0.001 % of total formulation |
| 6. | HFA134A OR HFA227 | q.s. |

### Process:

1) PVP was dissolved in PEG and part quantity ofHFA134A or HFA227.
2) The solution obtained in Step 1 was transferred to a mixing vessel.
3) Fluticasone furoate, Indacaterol and Glycopyrronium were homogenized with a part quantity of HFA.
4) The suspension obtained in step 3 was transferred to the mixing vessel where remaining quantity of HFA was added.
5) The resulting total suspension was mixed, recirculated and filled in into pre-crimped aluminum cans.

### Example 4

| **Sr. No.** | **Ingredients** | **Qty /Spray** |
|---|---|---|
| 1. | Fluticasone Furoate | 50 mcg |
| 2. | Glycopyrronium | 50 mcg |
| 3. | Indacaterol | 50 mcg |
| 4. | Ethanol | 15-20% of total formulation |
| 5. | Glycerol | 1% of total formulation |
| 6. | HCL(0.08N) | pH 2.5 - 3.5 |
| 7. | HFA134a | q.s. |

### Process:

1) Glycerol was dissolved in ethanol and required quantity of HCl was added.
2) Fluticasone furoate, Indacaterol and Glycopyrronium were dissolved in the solution obtained in step 1.
3) The resulting solution was transferred to the mixing vessel where HFA was added.
4) The resulting suspension was mixed, recirculated and filled in into pre-crimped aluminum cans.

### Example 5

| **Sr. No.** | **Ingredients** | **Qty /Spray** |
|---|---|---|
| 1. | Fluticasone Furoate | 50 mcg |
| 2. | Glycopyrronium | 50 mcg |
| 3. | Indacaterol | 50 mcg |
| 4. | Ethanol | 15-20% of total formulation |
| 5. | HCL(0.08N) | pH 2.5-3.5 |
| 6. | HFA134a | q.s. |

### Process:

1) Required quantity ofHCl was added to ethanol.
2) Fluticasone furoate, Indacaterol and Glycopyrronium were dissolved in the solution obtained in step 1.
3) The resulting solution was transferred to the mixing vessel where HFA was added.
4) The resulting suspension was mixed, recirculated and filled in into pre-crimped aluminum cans.

### Example 6

| **Sr. No.** | **Ingredients** | **Qty /Spray** |
|---|---|---|
| 1. | Fluticasone Furoate | 50 mcg |
| 2. | Glycopyrronium | 50 mcg |
| 3. | Indacaterol | 50 mcg |
| 4. | Ethanol | 15-20% of total formulation |
| 5. | Glycerol | 1% of total formulation |
| 6. | Citric acid anhydrous | pH 2.5 - 3.5 |
| 7. | HFA134a | q.s. |

### Process:

1) Citric acid anhydrous and glycerol were dissolved in ethanol.
2) Fluticasone furoate, Indacaterol and Glycopyrronium were dissolved in the solution obtained in step (1).
3) The solution obtained in step (2) was transferred to the main mixing vessel where it was mixed with entire quantity of HFA134a.
4) The resulting suspension was mixed, recirculated and filled in into pre-crimped aluminum cans.

### Example 7

| **Sr. No.** | **Ingredients** | **Qty /Spray** |
|---|---|---|
| 1. | Fluticasone Furoate | 50 mcg |
| 2. | Glycopyrronium | 50 mcg |
| 3. | Indacaterol | 50 mcg |
| 4. | Ethanol | 15-20% of total formulation |
| 5. | Citric acid anhydrous | pH 2.5 - 3.5 |
| 6. | HFA134a | q.s. |

### Process:

1) Citric acid anhydrous was dissolved in ethanol.
2) Fluticasone furoate, Indacaterol and Glycopyrronium were dissolved in the solution obtained in step (1).
3) The solution obtained in step (2) was transferred to the main mixing vessel where it was mixed with entire quantity of HFA134a.
4) The resulting suspension was mixed, recirculated and filled in into pre-crimped aluminum cans.

### Example 8

| **Sr. No.** | **Ingredients** | **Qty /Spray** |
|---|---|---|
| 1. | Fluticasone Furoate | 50 mcg |
| 2. | Glycopyrronium | 50 mcg |
| 3. | Indacaterol | 50 mcg |
| 4. | Ethanol | 1-2% of total formulation |
| 5. | Lecithin | 0.02 of the API |
| 6. | HFA134a or HFA227 | q.s. |

### Process:

1) Lecithin was dissolved in ethanol.
2) Glycopyrronium and Indacaterol were homogenized with part quantity of HFA and transferred to the mixing vessel.
3) Fluticasone furoate was homogenized with lecithin and ethanol.
4) The suspension obtained instep (3) was transferred to the main mixing vessel where the remaining quantity of HFA was added.
5) The resulting suspension was mixed, recirculated and filled in into pre-crimped aluminum cans.

### Example 9

| **Sr. No.** | **Ingredients** | **Qty /Spray** |
|---|---|---|
| 1. | Fluticasone Furoate | 50 mcg |
| 2. | Glycopyrronium | 50 mcg |
| 3. | Indacaterol | 50 mcg |
| 4. | Ethanol | 1-2% of total formulation |
| 5. | Oleic acid | 0.02 - 5% of the API |
| 6. | HFA134a or HFA227 | q.s. |

### Process:

1) Oleic acid was dissolved in ethanol.
2) Glycopyrronium and Indacaterol were homogenized with part quantity of HFA and transferred to the mixing vessel.
3) Fluticasone furoate was homogenized with oleic acid and ethanol.
4) The suspension obtained instep (3) was transferred to the main mixing vessel where the remaining quantity of HFA was added.
5) The resulting suspension was mixed, recirculated and filled in into pre-crimped aluminum cans.

### Example 10

| **Sr. No.** | **Ingredients** | **Qty / unit (mg)** |
|---|---|---|
| 1. | Glycopyrronium bromide | 0.050 |
| 2. | Indacaterol Maleate | 0.194 |
| 3. | Fluticasone Furoate | 0.100 |
| 4. | Lactose monohydrate IP/Ph.Eur/NF | 24.656 |
| | Total | 25.000 |

### Process:

1) Glycopyrronium, Indacaterol and Fluticasone furoate were sifted with a part quantity of lactose.
2) The co-sift of step 1 was then sifted with the remaining quantity of lactose and blended.
3) The blend of step 2 was then filled in capsules.

### Example 11

| **Sr. No.** | **Ingredients** | **Qty / unit (mg)** |
|---|---|---|
| 1. | Glycopyrronium bromide | 0.100 |
| 2. | Indacaterol Maleate | 0.194 |
| 3. | Fluticasone Furoate | 0.200 |
| 4. | Lactose monohydrate IP/Ph.Eur/NF | 24.506 |
| | Total | 25.000 |

### Process:

1) Glycopyrronium, Indacaterol and Fluticasone furoate were sifted with a part quantity of lactose.
2) The co-sift of step 1 was then sifted with the remaining quantity of lactose and blended.
3) The blend of step 2 was then filled in capsules.

### Example 12

| **Sr. No.** | **Ingredients** | **Qty / unit (mg)** |
|---|---|---|
| 1. | Glycopyrronium bromide | 0.200 |
| 2. | Indacaterol Maleate | 0.388 |
| 3. | Fluticasone Furoate | 0.400 |
| 4. | Lactose monohydrate IP/Ph.Eur/NF | 24.012 |
| | Total | 25.000 |

### Process:

1) Glycopyrronium, Indacaterol and Fluticasone furoate were sifted with a part quantity of lactose.
2) The co-sift of step 1 was then sifted with the remaining quantity of lactose and blended.
3) The blend of step 2 was then filled in capsules.

### Example 13

| **Sr. No.** | **Ingredients** | **Qty /Spray** |
|---|---|---|
| 1. | Fluticasone Furoate | 50 mcg |
| 2. | Glycopyrronium | 50 mcg |
| 3. | Formoterol | 24 mcg |
| 4. | HFA134A OR HFA227 | q.s |

### Process:

1) Fluticasone furoate, Glycopyrronium and Formoterol were homogenized with part quantity of HFA.
2) The suspension obtained in step 1 was transferred to the mixing vessel where remaining quantity of HFA was added.
3) The resulting suspension was mixed, recirculated and filled in into pre-crimped aluminum cans.

### Example 14

| **Sr. No.** | **Ingredients** | **Qty /Spray** |
|---|---|---|
| 1. | Fluticasone Furoate | 50 mcg |
| 2. | Glycopyrronium | 50 mcg |
| 3. | Formoterol | 24 mcg |
| 4. | Lactose | 100% of the drug |
| 5. | HFA134A OR HFA227 | q.s. |

### Process:

1) Fluticasone furoate, Formoterol and Glycopyrronium were homogenized with lactose and part quantity of HFA.
2) The suspension obtained in step 1 was transferred to the mixing vessel where remaining quantity of HFA was added.
3) The resulting suspension was mixed, recirculated and filled in into pre-crimped aluminum cans.

### Example 15

| **Sr. No.** | **Ingredients** | **Qty /Spray** |
|---|---|---|
| 1. | Fluticasone Furoate | 50 mcg |
| 2. | Glycopyrronium | 50 mcg |
| 3. | Formoterol | 24 mcg |
| 4. | PEG400/1000 | 0.3% of total formulation |
| 5. | PVP K 25 | 0.001 % of total formulation |
| 6. | HFA134A OR HFA227 | q.s. |

### Process:

1) PVP was dissolved in PEG and part quantity of HFA134A or HFA227.
2) The solution obtained in Step 1 was transferred to a mixing vessel.
3) Fluticasone furoate, Formoterol and Glycopyrronium were homogenized with a part quantity of HFA.
4) The suspension obtained in step 3 was transferred to the mixing vessel where remaining quantity of HFA was added.
5) The resulting total suspension was mixed, recirculated and filled in into pre-crimped aluminum cans.

### Example 16

| **Sr. No.** | **Ingredients** | **Qty /Spray** |
|---|---|---|
| 1. | Fluticasone Furoate | 50 mcg |
| 2. | Glycopyrronium | 50 mcg |
| 3. | Formoterol | 24 mcg |
| 4. | Ethanol | 15-20% of total formulation |
| 5. | Glycerol | 1% of total formulation |
| 6. | HCL(0.08N) | pH 2.5 - 3.5 |
| 7. | HFA134a | q.s. |

### Process:

1) Glycerol was dissolved in ethanol and required quantity of HCl was added.
2) Fluticasone furoate, Formoterol and Glycopyrronium were dissolved in the solution obtained in step 1.
3) The resulting solution was transferred to the mixing vessel where HFA was added.
4) The resulting suspension was mixed, recirculated and filled in into pre-crimped aluminum cans.

### Example 17

| **Sr. No.** | **Ingredients** | **Qty /Spray** |
|---|---|---|
| 1. | Fluticasone Furoate | 50 mcg |
| 2. | Glycopyrronium | 50 mcg |
| 3. | Formoterol | 24 mcg |
| 4. | Ethanol | 15-20% of total formulation |
| 5. | HCL(0.08N) | pH 2.5-3.5 |
| 6. | HFA134a | q.s. |

### Process:

1) Required quantity ofHCl was added to ethanol.
2) Fluticasone furoate, Formoterol and Glycopyrronium were dissolved in the solution obtained in step 1.
3) The resulting solution was transferred to the mixing vessel where HFA was added.
4) The resulting suspension was mixed, recirculated and filled in into pre-crimped aluminum cans.

### Example 18

| **Sr. No.** | **Ingredients** | **Qty /Spray** |
|---|---|---|
| 1. | Fluticasone Furoate | 50 mcg |
| 2. | Glycopyrronium | 50 mcg |
| 3. | Formoterol | 24 mcg |
| 4. | Ethanol | 1-2% of total formulation |
| 5. | Lecithin | 0.02 of the API |
| 6. | HFA134a or HFA227 | q.s. |

### Process:

1) Lecithin was dissolved in ethanol.
2) Glycopyrronium and Formoterol were homogenized with part quantity of HFA and transferred to the mixing vessel.
3) Fluticasoen furoate was homogenized with lecithin and ethanol.
4) The suspension obtained instep (3) was transferred to the main mixing vessel where the remaining quantity of HFA was added.
5) The resulting suspension was mixed, recirculated and filled in into pre-crimped aluminum cans.

### Example 19

| **Sr. No.** | **Ingredients** | **Qty /Spray** |
|---|---|---|
| 1. | Fluticasone Furoate | 50 mcg |
| 2. | Glycopyrronium | 50 mcg |
| 3. | Formoterol | 24 mcg |
| 4. | Ethanol | 1-2% of total formulation |
| 5. | Oleic acid | 0.02 - 5% of the API |
| 6. | HFA134a or HFA227 | q.s. |

### Process:

1) Oleic acid was dissolved in ethanol.
2) Glycopyrronium and Formoterol were homogenized with part quantity of HFA and transferred to the mixing vessel.
3) Fluticasone furoate was homogenized with oleic acid and ethanol.
4) The suspension obtained instep (3) was transferred to the main mixing vessel where the remaining quantity of HFA was added.
5) The resulting suspension was mixed, recirculated and filled in into pre-crimped aluminum cans.

### Example 20

| **Sr. No.** | **Ingredients** | **Qty / unit (mg)** |
|---|---|---|
| 1. | Glycopyrronium bromide | 0.050 |
| 2. | Formeterol Fumarate dihydrate | 0.006 |
| 3. | Fluticasone Furoate | 0.100 |
| 4. | Lactose monohydrate IP/Ph.Eur/NF | 24.844 |
| | Total | 25.000 |

### Process:

1) Glycopyrronium, Formoterol and Fluticasone furoate were sifted with a part quantity of lactose.
2) The co-sift of step 1 was then sifted with the remaining quantity of lactose and blended.
3) The blend of step 2 was then filled in capsules.

### Example 21

| **Sr. No.** | **Ingredients** | **Qty / unit (mg)** |
|---|---|---|
| 1. | Glycopyrronium bromide | 0.010 |
| 2. | Formeterol Fumarate dihydrate | 0.006 |
| 3. | Fluticasone Furoate | 0.200 |
| 4. | Lactose monohydrate IP/Ph.Eur/NF | 24.694 |
| | Total | 25.000 |

### Process:

1) Glycopyrronium, Formoterol and Fluticasone furoate were sifted with a part quantity of lactose.
2) The co-sift of step 1 was then sifted with the remaining quantity of lactose and blended.
3) The blend of step 2 was then filled in capsules.

### Example 22

| **Sr. No.** | **Ingredients** | **Qty / unit (mg)** |
|---|---|---|
| 1. | Glycopyrronium bromide | 0.200 |
| 2. | Formeterol Fumarate dihydrate | 0.012 |
| 3. | Fluticasone Furoate | 0.400 |
| 4. | Lactose monohydrate IP/Ph.Eur/NF | 24.388 |
| | Total | 25.000 |

### Process:

1) Glycopyrronium, Formoterol and Fluticasone furoate were sifted with a part quantity of lactose.
2) The co-sift of step 1 was then sifted with the remaining quantity of lactose and blended.
3) The blend of step 2 was then filled in capsules.

### Example 23

| **Sr. No.** | **Ingredients** | **Qty /Spray** |
|---|---|---|
| 1. | Fluticasone Furoate | 50 mcg |
| 2. | Glycopyrronium | 50 mcg |
| 3. | Vilanterol | 12.5 mcg |
| 4. | HFA134A OR HFA227 | q.s |

### Process:

1) Fluticasone furoate, Glycopyrronium and Vilanterol were homogenized with part quantity of HFA.
2) The suspension obtained in step 1 was transferred to the mixing vessel where remaining quantity of HFA was added.
3) The resulting suspension was mixed, recirculated and filled in into pre-crimped aluminum cans.

### Example 24

| **Sr. No.** | **Ingredients** | **Qty /Spray** |
|---|---|---|
| 1. | Fluticasone Furoate | 50 mcg |
| 2. | Glycopyrronium | 50 mcg |
| 3. | Vilanterol | 12.5 mcg |
| 4. | Lactose | 100% of the drug |
| 5. | HFA134A OR HFA227 | q.s. |

### Process:

1) Fluticasone furoate, Vilanterol and Glycopyrronium were homogenized with lactose and part quantity of HFA.
2) The suspension obtained in step 1 was transferred to the mixing vessel where remaining quantity of HFA was added.
3) The resulting suspension was mixed, recirculated and filled in into pre-crimped aluminum cans.

### Example 25

| **Sr. No.** | **Ingredients** | **Qty /Spray** |
|---|---|---|
| 1. | Fluticasone Furoate | 50 mcg |
| 2. | Glycopyrronium | 50 mcg |
| 3. | Vilanterol | 12.5 mcg |
| 4. | PEG400/1000 | 0.3% of total formulation |
| 5. | PVP K 25 | 0.001 % of total formulation |
| 6. | HFA134A OR HFA227 | q.s. |

### Process:

1) PVP was dissolved in PEG and part quantity of HFA134A or HFA227.
2) The solution obtained in Step 1 was transferred to a mixing vessel.
3) Fluticasone furoate, Vilanterol and Glycopyrronium were homogenized with a part quantity of HFA.
4) The suspension obtained in step 3 was transferred to the mixing vessel where remaining quantity of HFA was added.
5) The resulting total suspension was mixed, recirculated and filled in into pre-crimped aluminum cans.

### Example 26

| **Sr. No.** | **Ingredients** | **Qty /Spray** |
|---|---|---|
| 1. | Fluticasone Furoate | 50 mcg |
| 2. | Glycopyrronium | 50 mcg |
| 3. | Vilanterol | 12.5 mcg |
| 4. | Ethanol | 15-20% of total formulation |
| 5. | Glycerol | 1% of total formulation |
| 6. | HCL(0.08N) | pH 2.5 - 3.5 |
| 7. | HFA134a | q.s. |

### Process:

1) Glycerol was dissolved in ethanol and required quantity of HCl was added.
2) Fluticasone furoate, Vilanterol and Glycopyrronium were dissolved in the solution obtained in step 1.
3) The resulting solution was transferred to the mixing vessel where HFA was added.
4) The resulting suspension was mixed, recirculated and filled in into pre-crimped aluminum cans.

### Example 27

| **Sr. No.** | **Ingredients** | **Qty /Spray** |
|---|---|---|
| 1. | Fluticasone Furoate | 50 mcg |
| 2. | Glycopyrronium | 50 mcg |
| 3. | Vilanterol | 12.5 mcg |
| 4. | Ethanol | 15-20% of total formulation |
| 5. | HCL(0.08N) | pH 2.5-3.5 |
| 6. | HFA134a | q.s. |

### Process:

1) Required quantity of HCl was added to ethanol.
2) Fluticasone furoate, Vilanterol and Glycopyrronium were dissolved in the solution obtained in step 1.
3) The resulting solution was transferred to the mixing vessel where HFA was added.
4) The resulting suspension was mixed, recirculated and filled in into pre-crimped aluminum cans.

### Example 28

| **Sr. No.** | **Ingredients** | **Qty /Spray** |
|---|---|---|
| 1. | Fluticasone Furoate | 50 mcg |
| 2. | Glycopyrronium | 50 mcg |
| 3. | Vilanterol | 12.5 mcg |
| 4. | Ethanol | 1-2% of total formulation |
| 5. | Lecithin | 0.02 of the API |
| 6. | HFA134a or HFA227 | q.s. |

### Process:

1) Lecithin was dissolved in ethanol.
2) Glycopyrronium and Vilanterol were homogenized with part quantity of HFA and transferred to the mixing vessel.
3) Fluticasoen furoate was homogenized with lecithin and ethanol.
4) The suspension obtained instep (3) was transferred to the main mixing vessel where the remaining quantity of HFA was added.
5) The resulting suspension was mixed, recirculated and filled in into pre-crimped aluminum cans.

### Example 29

| **Sr. No.** | **Ingredients** | **Qty /Spray** |
|---|---|---|
| 1. | Fluticasone Furoate | 50 mcg |
| 2. | Glycopyrronium | 50 mcg |
| 3. | Vilanterol | 12.5 mcg |
| 4. | Ethanol | 1-2% of total formulation |
| 5. | Oleic acid | 0.02 - 5% of the API |
| 6. | HFA134a or HFA227 | q.s. |

### Process:

1) Oleic acid was dissolved in ethanol.
2) Glycopyrronium and Vilanterol were homogenized with part quantity of HFA and transferred to the mixing vessel.
3) Fluticasoen furoate was homogenized with oleic acid and ethanol.
4) The suspension obtained instep (3) was transferred to the main mixing vessel where the remaining quantity of HFA was added.
5) The resulting suspension was mixed, recirculated and filled in into pre-crimped aluminum cans.

### Example 30

| **Sr. No.** | **Ingredients** | **Qty / unit (mg)** |
|---|---|---|
| 1. | Glycopyrronium bromide | 0.050 |
| 2. | Vilanterol Trifenatate | 0.006 |
| 3. | Fluticasone Furoate | 0.100 |
| 4. | Lactose monohydrate IP/Ph.Eur/NF | 24.844 |
| | Total | 25.000 |

### Process:

1) Glycopyrronium, Vilanterol Trifenatate and Fluticasone furoate were sifted with a part quantity of lactose.
2) The co-sift of step 1 was then sifted with the remaining quantity of lactose and blended.
3) The blend of step 2 was then filled in capsules.

### Example 31

| **Sr. No.** | **Ingredients** | **Qty / unit (mg)** |
|---|---|---|
| 1. | Glycopyrronium bromide | 0.010 |
| 2. | Vilanterol Trifenatate | 0.025 |
| 3. | Fluticasone Furoate | 0.200 |
| 4. | Lactose monohydrate IP/Ph.Eur/NF | 24.675 |
| | Total | 25.000 |

### Process:

1) Glycopyrronium, Vilanterol Trifenatate and Fluticasone furoate were sifted with a part quantity of lactose.
2) The co-sift of step 1 was then sifted with the remaining quantity of lactose and blended.
3) The blend of step 2 was then filled in capsules.

### Example 32

| **Sr. No.** | **Ingredients** | **Qty / unit (mg)** |
|---|---|---|
| 1. | Glycopyrronium bromide | 0.200 |
| 2. | Vilanterol Trifenatate | 0.050 |
| 3. | Fluticasone Furoate | 0.400 |
| 4. | Lactose monohydrate IP/Ph.Eur/NF | 24.350 |
| | Total | 25.000 |

### Process:

1) Glycopyrronium, Vilanterol Trifenatate and Fluticasone furoate were sifted with a part quantity of lactose.
2) The co-sift of step 1 was then sifted with the remaining quantity of lactose and blended.
3) The blend of step 2 was then filled in capsules.

### Example 33

| **Sr. No.** | **Ingredients** | **Qty /Spray** |
|---|---|---|
| 1. | Fluticasone Furoate | 50 mcg |
| 2. | Glycopyrronium | 50 mcg |
| 3. | Olodaterol | 5 mcg |
| 4. | HFA134A OR HFA227 | q.s |

### Process:

1) Fluticasone furoate, Glycopyrronium and Olodaterol were homogenized with part quantity of HFA.
2) The suspension obtained in step 1 was transferred to the mixing vessel where remaining quantity of HFA was added.
3) The resulting suspension was mixed, recirculated and filled in into pre-crimped aluminum cans.

### Example 34

| **Sr. No.** | **Ingredients** | **Qty /Spray** |
|---|---|---|
| 1. | Fluticasone Furoate | 50 mcg |
| 2. | Glycopyrronium | 50 mcg |
| 3. | Olodaterol | 5 mcg |
| 4. | Lactose | 100% of the drug |
| 5. | HFA134A OR HFA227 | q.s. |

### Process:

1) Fluticasone furoate, Olodaterol and Glycopyrronium were homogenized with lactose and part quantity of HFA.
2) The suspension obtained in step 1 was transferred to the mixing vessel where remaining quantity of HFA was added.
3) The resulting suspension was mixed, recirculated and filled in into pre-crimped aluminum cans.

### Example 35

| **Sr. No.** | **Ingredients** | **Qty /Spray** |
|---|---|---|
| 1. | Fluticasone Furoate | 50 mcg |
| 2. | Glycopyrronium | 50 mcg |
| 3. | Olodaterol | 5 mcg |
| 4. | PEG400/1000 | 0.3% of total formulation |
| 5. | PVP K 25 | 0.001 % of total formulation |
| 6. | HFA134A OR HFA227 | q.s. |

### Process:

1) PVP was dissolved in PEG and part quantity of HFA134A or HFA227.
2) The solution obtained in Step 1 was transferred to a mixing vessel.
3) Fluticasone furoate, Olodaterol and Glycopyrronium were homogenized with a part quantity of HFA.
4) The suspension obtained in step 3 was transferred to the mixing vessel where remaining quantity of HFA was added.
5) The resulting total suspension was mixed, recirculated and filled in into pre-crimped aluminum cans.

### Example 36

| **Sr. No.** | **Ingredients** | **Qty /Spray** |
|---|---|---|
| 1. | Fluticasone Furoate | 50 mcg |
| 2. | Glycopyrronium | 50 mcg |
| 3. | Olodaterol | 5 mcg |
| 4. | Ethanol | 15-20% of total formulation |
| 5. | Glycerol | 1% of total formulation |
| 6. | HCL(0.08N) | pH 2.5 - 3.5 |
| 7. | HFA134a | q.s. |

### Process:

1) Glycerol was dissolved in ethanol and required quantity of HCl was added.
2) Fluticasone furoate, Olodaterol and Glycopyrronium were dissolved in the solution obtained in step 1.
3) The resulting solution was transferred to the mixing vessel where HFA was added.
4) The resulting suspension was mixed, recirculated and filled in into pre-crimped aluminum cans.

### Example 37

| **Sr. No.** | **Ingredients** | **Qty /Spray** |
|---|---|---|
| 1. | Fluticasone Furoate | 50 mcg |
| 2. | Glycopyrronium | 50 mcg |
| 3. | Olodaterol | 5 mcg |
| 4. | Ethanol | 15-20% of total formulation |
| 5. | HCL(0.08N) | pH 2.5-3.5 |
| 6. | HFA134a | q.s. |

### Process:

1) Required quantity of HCl was added to ethanol.
2) Fluticasone furoate, Olodaterol and Glycopyrronium were dissolved in the solution obtained in step 1.
3) The resulting solution was transferred to the mixing vessel where HFA was added.
4) The resulting suspension was mixed, recirculated and filled in into pre-crimped aluminum cans.

### Example 38

| **Sr. No.** | **Ingredients** | **Qty /Spray** |
|---|---|---|
| 1. | Fluticasone Furoate | 50 mcg |
| 2. | Glycopyrronium | 50 mcg |
| 3. | Olodaterol | 5 mcg |
| 4. | Ethanol | 1-2% of total formulation |
| 5. | Lecithin | 0.02 of the API |
| 6. | HFA134a or HFA227 | q.s. |

### Process:

1) Lecithin was dissolved in ethanol.
2) Glycopyrronium and Olodaterol were homogenized with part quantity of HFA and transferred to the mixing vessel.
3) Fluticasone furoate was homogenized with lecithin and ethanol.
4) The suspension obtained instep (3) was transferred to the main mixing vessel where the remaining quantity of HFA was added.
5) The resulting suspension was mixed, recirculated and filled in into pre-crimped aluminum cans.

### Example 39

| **Sr. No.** | **Ingredients** | **Qty /Spray** |
|---|---|---|
| 1. | Fluticasone Furoate | 50 mcg |
| 2. | Glycopyrronium | 50 mcg |
| 3. | Olodaterol | 5 mcg |
| 4. | Ethanol | 1-2% of total formulation |
| 5. | Oleic acid | 0.02 - 5% of the API |
| 6. | HFA134a or HFA227 | q.s. |

### Process:

1) Oleic acid was dissolved in ethanol.
2) Glycopyrronium and Olodaterol were homogenized with part quantity of HFA and transferred to the mixing vessel.
3) Fluticasone furoate was homogenized with oleic acid and ethanol.
4) The suspension obtained instep (3) was transferred to the main mixing vessel where the remaining quantity of HFA was added.
5) The resulting suspension was mixed, recirculated and filled in into pre-crimped aluminum cans.

### Example 40

| **Sr. No.** | **Ingredients** | **Qty / unit (mg)** |
|---|---|---|
| 1. | Glycopyrronium bromide | 0.050 |
| 2. | Olodaterol | 0.005 |
| 3. | Fluticasone Furoate | 0.100 |
| 4. | Lactose monohydrate IP/Ph.Eur/NF | 24.845 |
| | Total | 25.000 |

### Process:

1) Glycopyrronium, Olodaterol and Fluticasone furoate were sifted with a part quantity of lactose.
2) The co-sift of step 1 was then sifted with the remaining quantity of lactose and blended.
3) The blend of step 2 was then filled in capsules.

### Example 41

| **Sr. No.** | **Ingredients** | **Qty / unit (mg)** |
|---|---|---|
| 1. | Glycopyrronium bromide | 0.100 |
| 2. | Olodaterol | 0.005 |
| 3. | Fluticasone Furoate | 0.200 |
| 4. | Lactose monohydrate IP/Ph.Eur/NF | 23.695 |
| | Total | 25.000 |

### Process:

1) Glycopyrronium, Olodaterol and Fluticasone furoate were sifted with a part quantity of lactose.
2) The co-sift of step 1 was then sifted with the remaining quantity of lactose and blended.
3) The blend of step 2 was then filled in capsules.

### Example 42

| **Sr. No.** | **Ingredients** | **Qty / unit (mg)** |
|---|---|---|
| 1. | Glycopyrronium bromide | 0.200 |
| 2. | Olodaterol | 0.010 |
| 3. | Fluticasone Furoate | 0.400 |
| 4. | Lactose monohydrate IP/Ph.Eur/NF | 24.390 |
| | Total | 25.000 |

### Process:

1) Glycopyrronium, Olodaterol and Fluticasone furoate were sifted with a part quantity of lactose.
2) The co-sift of step 1 was then sifted with the remaining quantity of lactose and blended.
3) The blend of step 2 was then filled in capsules.

### Example 43

| **Sr. No.** | **Ingredients** | **Qty /Spray** |
|---|---|---|
| 1. | Mometasone furoate | 400 mcg |
| 2. | Glycopyrronium | 50 mcg |
| 3. | Olodaterol | 5 mcg |
| 4. | HFA134A OR HFA227 | q.s |

### Process:

1) Mometasone furoate, Glycopyrronium and Olodaterol were homogenized with part quantity of HFA.
2) The suspension obtained in step 1 was transferred to the mixing vessel where remaining quantity of HFA was added.
3) The resulting suspension was mixed, recirculated and filled in into pre-crimped aluminum cans.

### Example 44

| **Sr. No.** | **Ingredients** | **Qty /Spray** |
|---|---|---|
| 1. | Mometasone furoate | 400 mcg |
| 2. | Glycopyrronium | 50 mcg |
| 3. | Olodaterol | 5 mcg |
| 4. | Lactose | 100% of the drug |
| 5. | HFA134A OR HFA227 | q.s. |

### Process:

1) Mometasone furoate, Olodaterol and Glycopyrronium were homogenized with lactose and part quantity of HFA.
2) The suspension obtained in step 1 was transferred to the mixing vessel where remaining quantity of HFA was added.
3) The resulting suspension was mixed, recirculated and filled in into pre-crimped aluminum cans.

### Example 45

| **Sr. No.** | **Ingredients** | **Qty /Spray** |
|---|---|---|
| 1. | Mometasone furoate | 400 mcg |
| 2. | Glycopyrronium | 50 mcg |
| 3. | Olodaterol | 5 mcg |
| 4. | PEG400/1000 | 0.3% of total formulation |
| 5. | PVP K 25 | 0.001 % of total formulation |
| 6. | HFA134A OR HFA227 | q.s. |

### Process:

1) PVP was dissolved in PEG and part quantity of HFA134A or HFA227.
2) The solution obtained in Step 1 was transferred to a mixing vessel.
3) Mometasone furoate, Olodaterol and Glycopyrronium were homogenized with a part quantity of HFA.
4) The suspension obtained in step 3 was transferred to the mixing vessel where remaining quantity of HFA was added.
5) The resulting total suspension was mixed, recirculated and filled in into pre-crimped aluminum cans.

### Example 46

| **Sr. No.** | **Ingredients** | **Qty /Spray** |
|---|---|---|
| 1. | Mometasone furoate | 400 mcg |
| 2. | Glycopyrronium | 50 mcg |
| 3. | Olodaterol | 5 mcg |
| 4. | Ethanol | 15-20% of total formulation |
| 5. | Glycerol | 1% of total formulation |
| 6. | HCL(0.08N) | pH 2.5 - 3.5 |
| 7. | HFA134a | q.s. |

### Process:

1) Glycerol was dissolved in ethanol and required quantity of HCl was added.
2) Mometasone furoate, Olodaterol and Glycopyrronium were dissolved in the solution obtained in step 1.
3) The resulting solution was transferred to the mixing vessel where HFA was added.
4) The resulting suspension was mixed, recirculated and filled in into pre-crimped aluminum cans.

### Example 47

| **Sr. No.** | **Ingredients** | **Qty /Spray** |
|---|---|---|
| 1. | Mometasone furoate | 400 mcg |
| 2. | Glycopyrronium | 50 mcg |
| 3. | Olodaterol | 5 mcg |
| 4. | Ethanol | 15-20% of total formulation |
| 5. | HCL(0.08N) | pH 2.5-3.5 |
| 6. | HFA134a | q.s. |

### Process:

1) Required quantity ofHCl was added to ethanol.
2) Mometasone furoate, Olodaterol and Glycopyrronium were dissolved in the solution obtained in step 1.
3) The resulting solution was transferred to the mixing vessel where HFA was added.
4) The resulting suspension was mixed, recirculated and filled in into pre-crimped aluminum cans.

### Example 48

| **Sr. No.** | **Ingredients** | **Qty /Spray** |
|---|---|---|
| 1. | Mometasone furoate | 400 mcg |
| 2. | Glycopyrronium | 50 mcg |
| 3. | Olodaterol | 5 mcg |
| 4. | Ethanol | 1-2% of total formulation |
| 5. | Lecithin | 0.02 of the API |
| 6. | HFA134a or HFA227 | q.s. |

### Process:

1) Lecithin was dissolved in ethanol.
2) Glycopyrronium and Olodaterol were homogenized with part quantity of HFA and transferred to the mixing vessel.
3) Mometasone furoate was homogenized with lecithin and ethanol.
4) The suspension obtained instep (3) was transferred to the main mixing vessel where the remaining quantity of HFA was added.
5) The resulting suspension was mixed, recirculated and filled in into pre-crimped aluminum cans.

### Example 49

| **Sr. No.** | **Ingredients** | **Qty /Spray** |
|---|---|---|
| 1. | Mometasone furoate | 400 mcg |
| 2. | Glycopyrronium | 50 mcg |
| 3. | Olodaterol | 5 mcg |
| 4. | Ethanol | 1-2% of total formulation |
| 5. | Oleic acid | 0.02 - 5% of the API |
| 6. | HFA134a or HFA227 | q.s. |

### Process:

1) Oleic acid was dissolved in ethanol.
2) Glycopyrronium and Olodaterol were homogenized with part quantity of HFA and transferred to the mixing vessel.
3) Mometasone furoate was homogenized with oleic acid and ethanol.
4) The suspension obtained instep (3) was transferred to the main mixing vessel where the remaining quantity of HFA was added.
5) The resulting suspension was mixed, recirculated and filled in into pre-crimped aluminum cans.

### Example 50

| **Sr. No.** | **Ingredients** | **Qty** / **unit (mg)** |
|---|---|---|
| 1. | Glycopyrronium bromide | 0.200 |
| 2. | Olodaterol | 0.010 |
| 3. | Mometasone Furoate | 0.400 |
| 4. | Lactose monohydrate IP/Ph.Eur/NF | 24.390 |
| | Total | 25.000 |

### Process:

1) Glycopyrronium, Olodaterol and Mometasone furoate were sifted with a part quantity of lactose.
2) The co-sift of step 1 was then sifted with the remaining quantity of lactose and blended.
3) The blend of step 2 was then filled in capsules.

### Example 51

| **Sr. No.** | **Ingredients** | **Qty** / **unit (mg)** |
|---|---|---|
| 1. | Glycopyrronium bromide | 0.050 |
| 2. | Vilanterol Trifenatate | 0.006 |
| 3. | Lactose monohydrate IP/Ph.Eur/NF | 24.944 |
| | Total | 25.000 |

### Process:

1) Glycopyrronium and Vilanterol Trifenatate were sifted with a part quantity of lactose.
2) The co-sift of step 1 was then sifted with the remaining quantity of lactose and blended.
3) The blend of step 2 was then filled in capsules.

### Example 52

| **Sr. No.** | **Ingredients** | **Qty** / **unit (mg)** |
|---|---|---|
| 1. | Glycopyrronium bromide | 0.010 |
| 2. | Vilanterol Trifenatate | 0.025 |
| 3. | Lactose monohydrate IP/Ph.Eur/NF | 24.875 |
| | Total | 25.000 |

### Process:

1) Glycopyrronium and Vilanterol Trifenatate were sifted with a part quantity of lactose.
2) The co-sift of step 1 was then sifted with the remaining quantity of lactose and blended.
3) The blend of step 2 was then filled in capsules.

### Example 53

| **Sr. No.** | **Ingredients** | **Qty** / **unit (mg)** |
|---|---|---|
| 1. | Glycopyrronium bromide | 0.200 |
| 2. | Vilanterol Trifenatate | 0.050 |
| 3. | Lactose monohydrate IP/Ph.Eur/NF | 24.750 |
| | Total | 25.000 |

### Process:

1) Glycopyrronium and Vilanterol Trifenatate were sifted with a part quantity of lactose.
2) The co-sift of step 1 was then sifted with the remaining quantity of lactose and blended.
3) The blend of step 2 was then filled in capsules.

### Example 54

| **Sr. No.** | **Ingredients** | **Qty /Spray** |
|---|---|---|
| 1. | Glycopyrronium | 50 mcg |
| 2. | Vilanterol | 12.5 mcg |
| 3. | HFA134A OR HFA227 | q.s |

### Process:

1) Glycopyrronium and Vilanterol were homogenized with part quantity of HFA.
2) The suspension obtained in step 1 was transferred to the mixing vessel where remaining quantity of HFA was added.
3) The resulting suspension was mixed, recirculated and filled in into pre-crimped aluminum cans.

### Example 55

| **Sr. No.** | **Ingredients** | **Qty /Spray** |
|---|---|---|
| 1. | Glycopyrronium | 50 mcg |
| 2. | Vilanterol | 12.5 mcg |
| 3. | Lactose | 100% of the drug |
| 4. | HFA134A OR HFA227 | q.s. |

### Process:

1) Vilanterol and Glycopyrronium were homogenized with lactose and part quantity of HFA.
2) The suspension obtained in step 1 was transferred to the mixing vessel where remaining quantity of HFA was added.
3) The resulting suspension was mixed, recirculated and filled in into pre-crimped aluminum cans.

### Example 56

| **Sr. No.** | **Ingredients** | **Qty /Spray** |
|---|---|---|
| 1. | Glycopyrronium | 50 mcg |
| 2. | Vilanterol | 12.5 mcg |
| 3. | PEG400/1000 | 0.3% of total formulation |
| 4. | PVP K 25 | 0.001 % of total formulation |
| 5. | HFA134A OR HFA227 | q.s. |

### Process:

1) PVP was dissolved in PEG and part quantity of HFA134A or HFA227.
2) The solution obtained in Step 1 was transferred to a mixing vessel.
3) Vilanterol and Glycopyrronium were homogenized with a part quantity of HFA.
4) The suspension obtained in step 3 was transferred to the mixing vessel where remaining quantity of HFA was added.
5) The resulting total suspension was mixed, recirculated and filled in into pre-crimped aluminum cans.

### Example 57

| **Sr. No.** | **Ingredients** | **Qty /Spray** |
|---|---|---|
| 1. | Glycopyrronium | 50 mcg |
| 2. | Vilanterol | 12.5 mcg |
| 3. | Ethanol | 15-20% of total formulation |
| 4. | Glycerol | 1% of total formulation |
| 5. | HCL (0.08N) | pH 2.5 - 3.5 |
| 6. | HFA134a | q.s. |

### Process:

1) Glycerol was dissolved in ethanol and required quantity of HCl was added.
2) Vilanterol and Glycopyrronium were dissolved in the solution obtained in step 1.
3) The resulting solution was transferred to the mixing vessel where HFA was added.
4) The resulting suspension was mixed, recirculated and filled in into pre-crimped aluminum cans.

### Example 58

| **Sr. No.** | **Ingredients** | **Qty /Spray** |
|---|---|---|
| 1. | Glycopyrronium | 50 mcg |
| 2. | Vilanterol | 12.5 mcg |
| 3. | Ethanol | 15-20% of total |
| | | formulation |
| 4. | HCL (0.08N) | pH 2.5-3.5 |
| 5. | HFA134a | q.s. |

### Process:

1) Required quantity of HCl was added to ethanol.
2) Vilanterol and Glycopyrronium were dissolved in the solution obtained in step 1.
3) The resulting solution was transferred to the mixing vessel where HFA was added.
4) The resulting suspension was mixed, recirculated and filled in into pre-crimped aluminum cans.

### Example 59

| **Sr. No.** | **Ingredients** | **Qty /Spray** |
|---|---|---|
| 1. | Glycopyrronium | 50 mcg |
| 2. | Vilanterol | 12.5 mcg |
| 3. | Ethanol | 1-2% of total formulation |
| 4. | Lecithin | 0.02 of the API |
| 5. | HFA134a or HFA227 | q.s. |

### Process:

1) Lecithin was dissolved in ethanol.
2) Glycopyrronium and Vilanterol were homogenized with part quantity of HFA and transferred to the mixing vessel.
3) The solution obtained in step (1) was homogenized with part quantity of HFA
4) The mixture obtained in step (3) was transferred to the main mixing vessel where the remaining quantity of HFA was added.
5) The resulting suspension was mixed, recirculated and filled in into pre-crimped aluminum cans.

### Example 60

| **Sr. No.** | **Ingredients** | **Qty /Spray** |
|---|---|---|
| 1. | Glycopyrronium | 50 mcg |
| 2. | Vilanterol | 12.5 mcg |
| 3. | Ethanol | 1-2% of total formulation |
| 4. | Oleic acid | 0.02 - 5% of the API |
| 5. | HFA134a or HFA227 | q.s. |

### Process:

1) Oleic acid was dissolved in ethanol.
2) Glycopyrronium and Vilanterol were homogenized with part quantity of HFA and transferred to the mixing vessel.
3) The solution obtained in step (1) was homogenized with part quantity of HFA
4) The mixture obtained in step (3) was transferred to the main mixing vessel where the remaining quantity of HFA was added.
5) The resulting suspension was mixed, recirculated and filled in into pre-crimped aluminum cans.

### Example 61

| Sr. **No.** | **Ingredients** | **Qty / unit (mg)** |
|---|---|---|
| 1. | Glycopyrronium bromide | 0.100 |
| 2. | Olodaterol | 0.005 |
| 3. | Lactose monohydrate IP/Ph.Eur/NF | 24.944 |
| | Total | 25.000 |

### Process:

1) Glycopyrronium and Olodaterol were sifted with a part quantity of lactose.
2) The co-sift of step 1 was then sifted with the remaining quantity of lactose and blended.
3) The blend of step 2 was then filled in capsules.

### Example 62

| **Sr. No.** | **Ingredients** | **Qty** / **unit (mg)** |
|---|---|---|
| 1. | Glycopyrronium bromide | 0.200 |
| 2. | Olodaterol | 0.010 |
| 3. | Lactose monohydrate IP/Ph.Eur/NF | 24.875 |
| | Total | 25.000 |

### Process:

1) Glycopyrronium and Olodaterol were sifted with a part quantity of lactose.
2) The co-sift of step 1 was then sifted with the remaining quantity of lactose and blended.
3) The blend of step 2 was then filled in capsules.

### Example 63

| **Sr. No.** | **Ingredients** | **Qty /Spray** |
|---|---|---|
| 1. | Glycopyrronium | 100 mcg |
| 2. | Olodaterol | 5 mcg |
| 3. | HFA134A OR HFA227 | q.s |

### Process:

1) Glycopyrronium and Olodaterol were homogenized with part quantity of HFA.
2) The suspension obtained in step 1 was transferred to the mixing vessel where remaining quantity of HFA was added.
3) The resulting suspension was mixed, recirculated and filled in into pre-crimped aluminum cans.

### Example 64

| **Sr. No.** | **Ingredients** | **Qty /Spray** |
|---|---|---|
| 1. | Glycopyrronium | 100 mcg |
| 2. | Olodaterol | 5 mcg |
| 3. | Lactose | 100% of the drug |
| 4. | HFA134A OR HFA227 | q.s. |

### Process:

1) Olodaterol and Glycopyrronium were homogenized with lactose and part quantity of HFA.
2) The suspension obtained in step 1 was transferred to the mixing vessel where remaining quantity of HFA was added.
3) The resulting suspension was mixed, recirculated and filled in into pre-crimped aluminum cans.

### Example 65

| **Sr. No.** | **Ingredients** | **Qty /Spray** |
|---|---|---|
| 1. | Glycopyrronium | 100 mcg |
| 2. | Olodaterol | 5 mcg |
| 3. | PEG400/1000 | 0.3% of total formulation |
| 4. | PVP K 25 | 0.001 % of total formulation |
| 5. | HFA134A OR HFA227 | q.s. |

### Process:

1) PVP was dissolved in PEG and part quantity of HFA134A or HFA227.
2) The solution obtained in Step 1 was transferred to a mixing vessel.
3) Olodaterol and Glycopyrronium were homogenized with a part quantity of HFA.
4) The suspension obtained in step 3 was transferred to the mixing vessel where remaining quantity of HFA was added.
5) The resulting total suspension was mixed, recirculated and filled in into pre-crimped aluminum cans.

### Example 66

| **Sr. No.** | **Ingredients** | **Qty /Spray** |
|---|---|---|
| 1. | Glycopyrronium | 100 mcg |
| 2. | Olodaterol | 5 mcg |
| 3. | Ethanol | 15-20% of total formulation |
| 4. | Glycerol | 1% of total formulation |
| 5. | HCL (0.08N) | pH 2.5 - 3.5 |
| 6. | HFA134a | q.s. |

### Process:

1) Glycerol was dissolved in ethanol and required quantity of HCl was added.
2) Olodaterol and Glycopyrronium were dissolved in the solution obtained in step 1.
3) The resulting solution was transferred to the mixing vessel where HFA was added.
4) The resulting suspension was mixed, recirculated and filled in into pre-crimped aluminum cans.

### Example 67

| **Sr. No.** | **Ingredients** | **Qty /Spray** |
|---|---|---|
| 1. | Glycopyrronium | 100 mcg |
| 2. | Olodaterol | 5 mcg |
| 3. | Ethanol | 15-20% of total formulation |
| 4. | HCL (0.08N) | pH 2.5-3.5 |
| 5. | HFA134a | q.s. |

### Process:

1) Required quantity of HCl was added to ethanol.
2) Olodaterol and Glycopyrronium were dissolved in the solution obtained in step 1.
3) The resulting solution was transferred to the mixing vessel where HFA was added.
4) The resulting suspension was mixed, recirculated and filled in into pre-crimped aluminum cans.

### Example 68

| **Sr. No.** | **Ingredients** | **Qty /Spray** |
|---|---|---|
| 1. | Glycopyrronium | 100 mcg |
| 2. | Olodaterol | 5 mcg |
| 3. | Ethanol | 1-2% of total formulation |
| 4. | Lecithin | 0.02 of the API |
| 5. | HFA134a or HFA227 | q.s. |

### Process:

1) Lecithin was dissolved in ethanol.
2) Glycopyrronium and Olodaterol were homogenized with part quantity of HFA and transferred to the mixing vessel.
3) The solution obtained in step (1) was homogenized with part quantity of HFA
4) The mixture obtained in step (3) was transferred to the main mixing vessel where the remaining quantity of HFA was added.
5) The resulting suspension was mixed, recirculated and filled in into pre-crimped aluminum cans.

### Example 69

| **Sr. No.** | **Ingredients** | **Qty /Spray** |
|---|---|---|
| 1. | Glycopyrronium | 100 mcg |
| 2. | Olodaterol | 5 mcg |
| 3. | Ethanol | 1-2% of total formulation |
| 4. | Oleic acid | 0.02 - 5% of the API |
| 5. | HFA134a or HFA227 | q.s. |

### Process:

1) Oleic acid was dissolved in ethanol.
2) Glycopyrronium and Olodaterol were homogenized with part quantity of HFA and transferred to the mixing vessel.
3) The solution obtained in step (1) was homogenized with part quantity of HFA
4) The mixture obtained in step (3) was transferred to the main mixing vessel where the remaining quantity of HFA was added.
5) The resulting suspension was mixed, recirculated and filled in into pre-crimped aluminum cans.

### Example 70

| **Sr. No.** | **Ingredients** | **Qty** / **unit (mg)** |
|---|---|---|
| 1. | Glycopyrronium bromide | 0.100 |
| 2. | Carmoterol Hydrochloride | 0.002 |
| 3. | Lactose monohydrate IP/Ph.Eur/NF | 24.944 |
| | Total | 25.000 |

### Process:

1) Glycopyrronium and Carmoterol were sifted with a part quantity of lactose.
2) The co-sift of step 1 was then sifted with the remaining quantity of lactose and blended.
3) The blend of step 2 was then filled in capsules.

### Example 71

| **Sr. No.** | **Ingredients** | **Qty / unit (mg)** |
|---|---|---|
| 1. | Glycopyrronium bromide | 0.200 |
| 2. | Carmoterol Hydrochloride | 0.002 |
| 3. | Lactose monohydrate IP/Ph.Eur/NF | 24.875 |
| | Total | 25.000 |

### Process:

1) Glycopyrronium and Carmoterol were sifted with a part quantity of lactose.
2) The co-sift of step 1 was then sifted with the remaining quantity of lactose and blended.
3) The blend of step 2 was then filled in capsules.

### Example 72

| **Sr. No.** | **Ingredients** | **Qty /Spray** |
|---|---|---|
| 1. | Glycopyrronium | 100 mcg |
| 2. | Carmoterol | 2 mcg |
| 3. | HFA134A OR HFA227 | q.s |

### Process:

1) Glycopyrronium and Carmoterol were homogenized with part quantity of HFA.
2) The suspension obtained in step 1 was transferred to the mixing vessel where remaining quantity of HFA was added.
3) The resulting suspension was mixed, recirculated and filled in into pre-crimped aluminum cans.

### Example 73

| **Sr. No.** | **Ingredients** | **Qty /Spray** |
|---|---|---|
| 1. | Glycopyrronium | 100 mcg |
| 2. | Carmoterol | 2 mcg |
| 3. | Lactose | 100% of the drug |
| 4. | HFA134A OR HFA227 | q.s. |

### Process:

1) Carmoterol and Glycopyrronium were homogenized with lactose and part quantity of HFA.
2) The suspension obtained in step 1 was transferred to the mixing vessel where remaining quantity of HFA was added.
3) The resulting suspension was mixed, recirculated and filled in into pre-crimped aluminum cans.

### Example 74

| **Sr. No.** | **Ingredients** | **Qty /Spray** |
|---|---|---|
| 1. | Glycopyrronium | 100 mcg |
| 2. | Carmoterol | 2 mcg |
| 3. | PEG400/1000 | 0.3% of total formulation |
| 4. | PVP K 25 | 0.001 % of total formulation |
| 5. | HFA134A OR HFA227 | q.s. |

### Process:

1) PVP was dissolved in PEG and part quantity of HFA134A or HFA227.
2) The solution obtained in Step 1 was transferred to a mixing vessel.
3) Carmoterol and Glycopyrronium were homogenized with a part quantity of HFA.
4) The suspension obtained in step 3 was transferred to the mixing vessel where remaining quantity of HFA was added.
5) The resulting total suspension was mixed, recirculated and filled in into pre-crimped aluminum cans.

### Example 75

| **Sr. No.** | **Ingredients** | **Qty /Spray** |
|---|---|---|
| 1. | Glycopyrronium | 100 mcg |
| 2. | Carmoterol | 2 mcg |
| 3. | Ethanol | 15-20% of total formulation |
| 4. | Glycerol | 1% of total formulation |
| 5. | HCL (0.08N) | pH 2.5 - 3.5 |
| 6. | HFA134a | q.s. |

### Process:

1) Glycerol was dissolved in ethanol and required quantity of HCl was added.
2) Carmoterol and Glycopyrronium were dissolved in the solution obtained in step 1.
3) The resulting solution was transferred to the mixing vessel where HFA was added.
4) The resulting suspension was mixed, recirculated and filled in into pre-crimped aluminum cans.

### Example 76

| **Sr. No.** | **Ingredients** | **Qty /Spray** |
|---|---|---|
| 1. | Glycopyrronium | 100 mcg |
| 2. | Carmoterol | 2 mcg |
| 3. | Ethanol | 15-20% of total formulation |
| 4. | HCL (0.08N) | pH 2.5-3.5 |
| 5. | HFA134a | q.s. |

### Process:

1) Required quantity of HCl was added to ethanol.
2) Carmoterol and Glycopyrronium were dissolved in the solution obtained in step 1.
3) The resulting solution was transferred to the mixing vessel where HFA was added.
4) The resulting suspension was mixed, recirculated and filled in into pre-crimped aluminum cans.

### Example 77

| **Sr. No.** | **Ingredients** | **Qty /Spray** |
|---|---|---|
| 1. | Glycopyrronium | 100 mcg |
| 2. | Carmoterol | 2 mcg |
| 3. | Ethanol | 1-2% of total formulation |
| 4. | Lecithin | 0.02 of the API |
| 5. | HFA134a or HFA227 | q.s. |

### Process:

1) Lecithin was dissolved in ethanol.
2) Glycopyrronium and Carmoterol were homogenized with part quantity of HFA and transferred to the mixing vessel.
3) The solution obtained in step (1) was homogenized with part quantity of HFA
4) The mixture obtained in step (3) was transferred to the main mixing vessel where the remaining quantity of HFA was added.
5) The resulting suspension was mixed, recirculated and filled in into pre-crimped aluminum cans.

### Example 78

| **Sr. No.** | **Ingredients** | **Qty /Spray** |
|---|---|---|
| 1. | Glycopyrronium | 100 mcg |
| 2. | Carmoterol | 2 mcg |
| 3. | Ethanol | 1-2% of total formulation |
| 4. | Oleic acid | 0.02 - 5% of the API |
| 5. | HFA134a or HFA227 | q.s. |

### Process:

1) Oleic acid was dissolved in ethanol.
2) Glycopyrronium and Carmoterol were homogenized with part quantity of HFA and transferred to the mixing vessel.
3) The solution obtained in step (1) was homogenized with part quantity of HFA
4) The mixture obtained in step (3) was transferred to the main mixing vessel where the remaining quantity of HFA was added.
5) The resulting suspension was mixed, recirculated and filled in into pre-crimped aluminum cans.

It will be readily apparent to one skilled in the art that varying substitutions and modifications may be made to the invention disclosed herein without departing from the spirit of the invention. Thus, it should be understood that although the present invention has been specifically disclosed by the preferred embodiments and optional features, modification and variation of the concepts herein disclosed may be resorted to by those skilled in the art, and such modifications and variations are considered to be falling within the scope of the invention.

It is to be understood that the phraseology and terminology used herein is for the purpose of description and should not be regarded as limiting. The use of "including," "comprising," or "having" and variations thereof herein is meant to encompass the items listed thereafter and equivalents thereof as well as additional items.

It must be noted that, as used in this specification and the appended claims, the singular forms "a," "an" and "the" include plural references unless the context clearly dictates otherwise. Thus, for example, reference to "an excipient" includes a single excipient as well as two or more different excipients, and the like.

The Appendix comprises the claims of the parent application set out as clauses, and these are included here to preserve all subject matter. They represent additional embodiments of the present invention and may form the basis of subsequent claims.

### APPENDIX

1. A pharmaceutical composition comprising glycopyrrolate in combination with a beta₂-agonist selected from indacaterol, formoterol, vilanterol, carmoterol olodaterol, optionally, one or more pharmaceutically acceptable excipients.
2. A pharmaceutical composition according to clause 1, wherein the beta₂-agonist is indacaterol.
3. A pharmaceutical composition according to clause 2 or 3, wherein indacaterol is an amount ranging from 25-800mcg.
4. A pharmaceutical composition according to clause 1, wherein the beta₂-agonist is formoterol.
5. A pharmaceutical composition according to clause 4, wherein formoterol is an amount ranging from 12-24mcg.
6. A pharmaceutical composition according to clause 1, wherein the beta₂-agonist is vilanterol.
7. A pharmaceutical composition according to clause 6, wherein vilanterol is an amount ranging from 3-50mcg.
8. A pharmaceutical composition according to clause 1, wherein the beta₂-agonist is carmoterol.
9. A pharmaceutical composition according to clause 8, wherein carmoterol is an amount ranging from 1-4mcg.
10. A pharmaceutical composition according to clause 8 or 9, wherein the carmoterol is in the form of carmoterol hydrochloride.
11. A pharmaceutical composition according to clause 1, wherein the beta₂-agonist is olodaerol.
12. A pharmaceutical composition according to clause 11, wherein olodaterol is an amount ranging from 3-50mcg.
13. A pharmaceutical composition according to any of the preceding clauses, comprising glycopyrrolate and vilanterol.
14. A pharmaceutical composition according to any of the preceding clauses comprising glycopyrrolate and carmoterol.
15. A pharmaceutical composition according to any of the preceding clauses, comprising glycopyrrolate and olodaterol.
16. A pharmaceutical composition comprising in combination glycopyrrolate in combination with a beta₂-agonist, and one or more corticosteroid, optionally with one or more pharmaceutically acceptable excipients.
17. A pharmaceutical composition according to clause, wherein the beta₂-agonist is selected from indacaterol, formoterol, vilanterol, carmoterol olodaterol.
18. A pharmaceutical composition according to clause 17, wherein the corticosteroid is selected from fluticasone, mometasone.
19. A pharmaceutical composition according to clause 16 or 18, wherein the corticosteroid is fluticasone.
20. A pharmaceutical composition according to clause 16, 18 or 19, wherein the fluticasone is in the form of an ester of fluticasone.
21. A pharmaceutical composition according to clause 16, 18 or 20, wherein the fluticasone is present in an amount ranging from 25 -800mcg.
22. A pharmaceutical composition according to clause 19, 20 or 21, wherein the fluticasone is in the form of fluticasone furoate.
23. A pharmaceutical composition according to clause 16 or 18, wherein the corticosteroid is mometasone.
24. A pharmaceutical composition according to clause 16, 18 or 23, wherein the mometasone is in the form of an ester of mometasone
25. A pharmaceutical composition according to clause 16, 18 or 24, wherein the mometasone is present in an amount ranging from 400-800mcg.
26. A pharmaceutical composition according to any one of the preceding clauses, wherein glycopyrrolate is present in an amount ranging from 0.5-10mcg.
27. A pharmaceutical composition according to any of the preceding clauses, comprising glycopyrrolate indacaterol and fluticasone furoate.
28. A pharmaceutical composition according to any of the preceding clauses, comprising glycopyrrolate, indacaterol and fluticasone furoate.
29. A pharmaceutical composition according to any of the preceding clauses, comprising glycopyrrolate, formoterol and fluticasone furoate.
30. A pharmaceutical composition according to any of the preceding clauses, comprising glycopyrrolate, vilanterol and fluticasone furoate.
31. A pharmaceutical composition according to any of the preceding clauses, comprising glycopyrrolate, olodaterol and fluticasone furoate.
32. A pharmaceutical composition according to any of the preceding clauses, comprising glycopyrrolate, olodaterol and mometasone.
33. A pharmaceutical composition according to any one of the preceding clauses wherein pharmaceutical composition along with any excipients are formulated in a single pharmaceutical composition
34. A pharmaceutical composition according to any one of the preceding clauses, formulated as a composition for inhalation.
35. A pharmaceutical composition according to claim 34, formulated as a composition for inhalation in the form of a metered dose inhaler (MDI), dry powder inhaler (DPI), nebulizer, nasal spray, nasal drops or an insufflation powder.
36. A pharmaceutical composition according to any one of clauses 1 to 35, formulated for use in a metered dose inhaler.
37. A pharmaceutical composition according to clause 34, 35 or 36, further comprising a propellant.
38. A pharmaceutical composition according to clause 34, 35, 36 or 37, further comprising an excipient selected from a cosolvent, an antioxidant, a surfactant, a bulking agent and a lubricant.
39. A pharmaceutical composition according to any one of clauses 1 to 35, formulated for use as a dry powder inhalation formulation.
40. A pharmaceutical composition according to clause 39, further comprising at least one finely divided pharmaceutically acceptable carrier suitable for use in dry powder inhalation formulations.
41. A combination composition according to clause 40, wherein said carrier includes a saccharide and/or a sugar alcohol.
42. A combination composition according to any one of clauses 1 to 35, formulated for use as an inhalation solution/suspension.
43. A combination composition according to clause 42, further comprising an excipient selected from a wetting agent, osmotic agent, a pH regulator, a buffering agent and a complexing agent, provided in a pharmaceutically acceptable vehicle.
44. A pharmaceutical composition according to any one of the preceding clauses for once daily administration.
45. A process for manufacturing a pharmaceutical composition according to according to any one of clauses 1 to 44, comprising combining glycopyrrolate with a beta₂-agonist selected from indacaterol, formoterol, vilanterol, carmoterol olodaterol, and optionally, one or more pharmaceutically acceptable excipients.
46. A process for manufacturing a pharmaceutical composition according to any one of clauses 1 to 44 comprising combining glycopyrrolate with a beta₂-agonist selected from indacaterol, formoterol, vilanterol, carmoterol olodaterol, and corticosteroid is selected from fluticasone, mometasone, optionally, one or more pharmaceutically acceptable excipients.
47. The use of glycopyrrolate with a beta₂-agonist selected from indacaterol, formoterol, vilanterol, carmoterol olodaterol, in the manufacture of a medicament for the prophylaxis or treatment of a respiratory, inflammatory or obstructive airway disease.
48. The use of glycopyrrolate with a beta₂-agonist selected from indacaterol, formoterol, vilanterol, carmoterol olodaterol, and corticosteroid is selected from fluticasone, mometasone, in the manufacture of a medicament for the prophylaxis or treatment of a respiratory, inflammatory or obstructive airway disease.
49. The use according to clause 48 wherein the fluticasone is provided in the form of fluticasone furoate
50. The use according to clause 47, 48 or 49, wherein said medicament is for once daily administration.
51. The use according to clause 47, 48, 49 or 50 wherein the disease is COPD or asthma.
52. A method of prophylaxis or treatment of a respiratory, inflammatory or obstructive airway disease, comprising administering a therapeutically effective amount of a pharmaceutical composition according to any one of clauses 1 to 44 to a patient in need thereof.
53. A method according to clause, wherein said pharmaceutical composition is administered once daily.
54. A method according to clause 52, or 53, wherein the disease is COPD or asthma.
55. A pharmaceutical composition substantially as herein described with reference to the examples.
56. A process for making a pharmaceutical composition substantially as herein described with reference to the examples.

## Claims

1. A pharmaceutical composition comprising glycopyrronium in combination with vilanterol, optionally with one or more pharmaceutically acceptable excipients.

2. A pharmaceutical composition according to claim 1, wherein glycopyrronium and/or vilanterol are in the form of their pharmaceutically acceptable salts, esters, solvates, hydrates, enantiomers, derivatives, polymorphs, prodrugs.

3. A pharmaceutical composition according to claim 1 or 2, wherein vilanterol is present in an amount ranging from 3-50 mcg.

4. A pharmaceutical composition according to claim 1, 2 or 3 wherein vilanterol is present as vilanterol trifenatate.

5. A pharmaceutical composition according to any preceding claim, wherein glycopyrronium is present in an amount ranging from 50-200 mcg.

6. A pharmaceutical composition according to any preceding claim, further comprising fluticasone, preferably fluticasone propionate or fluticasone furoate.

7. A pharmaceutical composition according to any preceding claim wherein fluticasone is present in an amount ranging from 25 -800 mcg.

8. A pharmaceutical composition according to any preceding claim, in the form of separate formulations or a single combined formulation.

9. A pharmaceutical composition according to any preceding claim, formulated as a composition for inhalation.

10. A pharmaceutical composition according to claim 9, formulated as a composition for inhalation in the form of a metered dose inhaler (MDI), dry powder inhaler (DPI), nebulizer, nasal spray, nasal drops or an insufflation powder.

11. A pharmaceutical composition according to any preceding claim for once daily administration.

12. A process for manufacturing a pharmaceutical composition according to any one of claims 1 to 11 comprising combining glycopyrronium with vilanterol, optionally, with fluticasone and one or more pharmaceutically acceptable excipients.

13. Use of glycopyrronium in combination with vilanterol for the prophylaxis or treatment of a respiratory, inflammatory or obstructive airway disease.

14. Use according to claim 13, further comprising fluticasone.

15. Use according to claim 13 or 14, wherein the disease is COPD or asthma.
